# FASCICULE DE BREVET EUROPEEN

(11) **EP 0 422 090 B1**
(45) Date de publication et mention de la délivrance du brevet: **02.06.1993**
(21) Numéro de dépôt: 89907771.3
(22) Date de dépôt: 19.06.1989
(51) Int. Cl.: C07H 19/073, C07H 21/04, C12Q 1/68

(54) **DERIVES DE NUCLEOSIDES UTILISABLES POUR LA SYNTHESE D'OLIGONUCLEOTIDES MARQUES, OLIGONUCLEOTIDES OBTENUS A PARTIR DE CES DERIVES ET LEUR SYNTHESE**
NUKLEOSIDE DERIVATE, NÜTZLICH FÜR DIE SYNTHESE VON MARKIERTEN OLIGONUKLEOTIDEN, MIT DIESEN DERIVATEN HERGESTELLTE OLIGONUKLEOTIDE UND IHRE SYNTHESE
DERIVATIVES OF NUCLEOSIDES WHICH MAY BE USED FOR THE SYNTHESIS OF MARKED OLIGONUCLEOTIDES, AND OLIGONUCLEOTIDES OBTAINED FROM THESE DERIVATIVES AND THEIR SYNTHESIS

(30) Priorité: 20.06.1988 FR 8808240
(43) Date de publication de la demande: 17.04.1991
(73) Titulaire: CIS BIO INTERNATIONAL, F-91000 Saclay (FR)
(72) Inventeur: ROGET, André, F-38600 Fontaine (FR); TEOULE, Robert, F-38000 Grenoble (FR); BAZIN, Hervé, F-38100 Grenoble (FR)
(74) Mandataire: Des Termes, Monique
(86) Numéro de dépôt international: FR8900312
(87) Numéro de publication internationale: WO8912642

(56) Documents cités:
- EP-A- 0 063 879
- WO-A- /01941
- WO-A-84/03285
- WO-A-86/06726
- WO-A-88/00593
- NUCLEIC ACIDS RESEARCH, vol. 16, no. 9, 1988; COOK et al., pp. 4077-4095#
- NUCLEIC ACIDS RESEARCH, vol. 17; SPROAT et al., pp. 3373-3383#
- NUCLEIC ACIDS RESEARCH, vol. 17, no. 19, 1989; pp. 7643-7651#

## Description

L'invention a pour objet des dérivés de nucléosides utilisables pour la synthèse d'oligonucléotides marqués, les oligonucléotides obtenus à partir de ces dérivés et leur synthèse.

Les oligonucléotides marqués, par exemple les sondes d'acides nucléiques, constituent un outil de diagnostic très intéressant dans différents domaines tels que les maladies génétiques, les maladies infectieuses, le cancer et le typage cellulaire.

Ces techniques de diagnostic sont basées sur l'utilisation de l'hybridation, c'est-à-dire l'association par des liaisons hydrogène de deux brins d'acides nucléiques (ADN ou ARN) complémentaires. Cette réaction peut ainsi mettre en jeu deux chaînes d'ADN, une chaîne d'ADN et une chaîne d'ARN, ou bien encore deux chaînes d'ARN. Lors de cette réaction, la thymine (ou l'uracile pour l'ARN) se place en face de l'adénine et la cytosine se place en face de la guanine. La formation de cette double hélice dépend de facteurs physico-chimiques tels que le pH, la concentration en ions sodium, la température et la durée de la réaction. Dans les techniques de diagnostic, on utilise une sonde oligonucléotidique présentant une séquence complémentaire à celle de la cible, c'est-à-dire de l'échantillon biologique à déterminer. De plus, pour permettre la détection de la sonde, celle-ci comporte un élément marqueur approprié susceptible d'être détecté par des méthodes telles que les méthodes radioactives, immunoenzymatiques, colorimétriques, fluorimétriques ou luminescentes.

Lorsqu'on utilise des éléments marqueurs non radioactifs, on peut préparer directement les sondes nucléotidiques marquées bien avant leur utilisation par des procédés biochimiques ou chimiques.

En revanche, lorsque l'élément marqueur est radioactif, l'introduction de cet élément dans la sonde doit être effectuée extemporanément.

Parmi les procédés biochimiques de production d'oligonucléotides marqués, on connaît par Langer et al (Proc. Natl. Acad. Sci. USA Vol. 78, n° 11, p. 6633-6637, novembre 1981, Biochemistry), un procédé de marquage de polynucléotides par la biotine utilisant diverses ADN ou ARN polymérases et un dérivé de nucléoside comportant une partie uracile sur laquelle est fixée la biotine par l'intermédiaire d'un maillon allylamine. Cette méthode présente l'inconvénient majeur de ne pas être applicable au marquage de sondes courtes.

Murasugi et al dans DNA, vol. 3, n°3, 1984, p. 269-277, ont décrit la préparation de sondes oligonucléotidiques marquées à la biotine par réaction de deux oligonucléotides et de trois dérivés de nucléosides dont le dérivé biotinylé de l'uridine triphosphate, en présence d'ADN polymérase I de Escherichia coli. Dans ce cas, il est donc nécessaire d'utiliser deux oligonucléotides dont l'un en fort excès. D'autre part, le rendement de marquage est faible (10 à 30%) pour incorporer une seule biotine dans la sonde oligonucléotidique.

Le document EP-A- 063 879 (Yale University) décrit des dérivés de nucléosides comportant une purine substituée en position 8 ou une pyrimidine substituée en position 5 par un radical capable de former un complexe détectable avec un polypeptide, qui peut être utilisé pour la synthèse d'oligonucléotides par polymérisation enzymatique.

Le document WO-A- 880 593 (Institut Pasteur) décrit un dérivé de désoxy-2' adénosine biotinylé en position 8 de l'adénosine, qui peut être aussi incorporé enzymatiquement dans un oligonucléotide.

Comme précédemment, ceci ne permet le marquage de l'oligonucléotide qu'en position 3'. Par ailleurs, ce dérivé ne peut être utilisé dans les appareillages automatiques de synthèse d'oligonucléotides.

L'incorporation de nucléotides biotinylés a été aussi réalisée suivant la méthode décrite par WU dans Methods in Enzymology, vol. 65, 1980, p. 43-62, en utilisant la terminale désoxynucléotidyl transférase. Cette méthode est intéressante car elle permet d'incorporer plusieurs biotines (3 à 5 généralement) sur une sonde oligonucléotidique courte. Ce marquage nécessite une étape supplémentaire après la synthèse de l'oligonucléotide. Il est difficile de préparer une sonde comportant un nombre défini de marqueurs parce qu'on obtient un mélange complexe de produits de différentes tailles qu'il faudrait séparer pour avoir une sonde de point de fusion bien défini. Il semble beaucoup plus difficile de réaliser un double marquage. Par ailleurs, le marquage s'effectue uniquement en 3'.

Parmi les méthodes chimiques, on connait une méthode dans laquelle le marquage est effectué en bout de chaîne après synthèse et déprotection de l'oligonucléotide. Cette méthode consiste à fixer l'élément marqueur sur la partie osidique de l'oligonucléotide, comme il est décrit par A. Chollet et al dans Nucleic Acids Research, vol. 13, n°5, 1985, p. 1529-1540. L'utilisation de cette méthode présente l'inconvénient majeur de conduire à la fixation d'une seule molécule d'élément marqueur par oligonucléotide et de nécessiter, après la synthèse oligonucléotidique, des étapes additionnelles de réactions chimiques pour fixer cet élément marqueur.

Afin d'augmenter le nombre d'éléments marqueurs fixés sur l'oligonucléotide, on a envisagé de fixer cet élément marqueur sur les bases de l'oligonucléotide, après synthèse et déprotection de celui-ci comme il est décrit par Roduit et al, dans Nucleosides & Nucleotides, 6 (1 & 2), 349-352 (1987). Dans ce cas, on peut fixer plusieurs éléments marqueurs, mais il est impossible d'utiliser des marqueurs différents sur certains nucléotides de la chaîne oligonucléotidique. Par ailleurs, le rendement de marquage reste inférieur à 60%.

Une autre méthode de production d'oligonucléotides marqués par la biotine est décrite par Kierzek et al, dans Nucleosides & nucleotides, 6(1 & 2), 403-405 (1987) et dans WO-A- 86 06726. Dans ce cas on réalise la synthèse de l'oligonucléotide en utilisant les dérivés des nucléosides ayant les groupements fonctionnels utilisés en synthèse oligonucléotidique ainsi que des dérivés de nucléosides ayant un maillon -(CH₂)₆-NH-R (R représentant un groupement protecteur tel que le groupe trifluoracétyle) fixé sur la cytidine. A la fin de la synthèse, et après avoir réalisé la déprotection, on fixe la biotine sur ces maillons de la cytidine. Ainsi que dans les publications citées précédemment, il est impossible d'utiliser des marqueurs différents. De même, il est nécessaire d'effectuer diverses réactions chimiques après la synthèse et la déprotection de l'oligonucléotide, ce qui ne peut être réalisé de façon automatique dans les appareils de synthèse d'oligonucléotides.

Le document WO-A- 8403285 illustre une méthode de production d'oligonucléotides marqués analogue dans laquelle on part de dérivés de nucléosides dont les bases comportent des substituants capables de réagir avec un élément marqueur, et on effectue le marquage après synthèse de l'oligonucléotide.

Le document WO 89/01941 décrit aussi un procédé de production d'oligonucléotides marqués, qui consiste à incorporer des thymidines activées à la place des cytidines, lors de la synthèse chimique en phase solide de la séquence oligonucléotidique, et à faire réagir ensuite la séquence oligonucléotidique avec un composé de formule R-A-R ' dans laquelle R et R' représentent NH₂, SH ou OH où R' représente NHX, SX, ou OX avec X étant un groupe capable de servir de marqueur. Par réaction de la séquence avec le composé RAR', on transforme les thymines activées de la séquence en méthyl-5-cytosines avec une chaîne de substitution en position 4 qui peut être marquée ou qui, lorsqu'elle n'est pas marquée, peut être transformée par introduction d'un groupe marqueur tel que la biotine.

Aussi, comme l'indique l'article de Cook et al, publié récemment dans Nucleic Acids Research, vol. 16, n° 9, 1988, p. 4077-4095, bien qu'il existe deux approches possibles qui sont d'introduire la biotine, soit avant, soit après la synthèse oligonucléotidique, il est recommandé de réaliser le marquage après la synthèse en raison des réactions indésirables susceptibles de se produire entre la biotine et les réactifs utilisés pour la synthèse oligonucléotidique.

Ainsi, les procédés connus actuellement de production d'oligonucléotides marqués ne permettent pas d'introduire dans de bonnes conditions une ou plusieurs molécules de marqueurs à des emplacements parfaitement définis dans la channe de l'oligonucléotide, en particulier en position 5, et de réaliser un marquage de la channe par des marqueurs différents. Par ailleurs, les procédés connus ont l'inconvénient de nécessiter pour la plupart des opérations complémentaires après la synthèse proprement dite de l'oligonucléotide, ce qui ne permet pas d'effectuer automatiquement la synthèse directe d'oligonucléotides marqués.

La présente invention a précisément pour objet des dérivés de nucléosides utilisables pour la synthèse d'oligonucléotides marqués, qui permettent de pallier les inconvénients des procédés précités.

Selon l'invention, le dérivé de nucléoside utilisable pour la synthèse d'oligonucléotides marqués, répond à la formule : 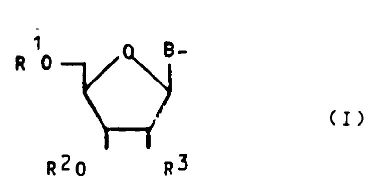
dans laquelle :
- R¹ représente un atome d'hydrogène, ou un radical labile en milieu acide convenant à la synthèse nucléotidique,
- R² représente un atome d'hydrogéne, un radical protecteur, un radical fonctionnel adapté à la synthèse oligonucléotidique, ou un support utilisable pour la synthèse oligonucléotidique comportant un bras espaceur de liaison avec l'atome d'oxygène,
- R³ représente un atome d'hydrogène, le radical hydroxyle ou un radical hydroxyle protégé,
- B représente un radical dérivé d'une base purique ou pyrimidique de formule : 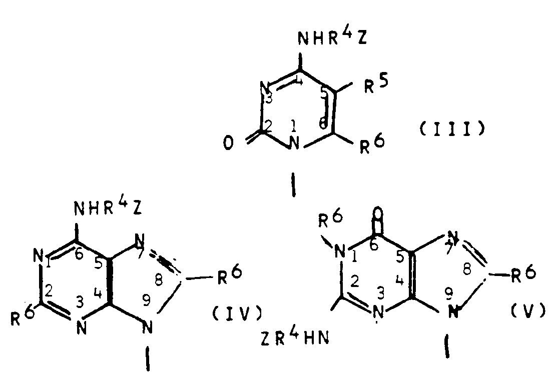 dans lesquelles R⁵ représente H, CH₃ ou R⁴Z, R⁶ représente un atome d'hydrogène ou le groupement R⁴Z, et le radical de formule B est relié par le N en position 1 du radical de formule III ou en position 9 des radicaux de formule IV et V à la partie osidique,
- R⁴ représente une simple liaison ou un radical hydrocarboné choisi parmi les radicaux de formules :
   -NH(CH₂)ₙ-X-(CH₂)ₚ-
   -NH-(CH₂)ₙ-X-(CH₂)ₚ-NH-
   -O-(CH₂)ₙ-X-(CH₂)ₚ-
   -O-(CH₂)ₙ-X-(CH₂)ₚ-NH-
   -S-(CH₂)ₙ-X-(CH₂)ₚ-
   -S-(CH₂)ₙ-X-(CH₂)ₚ-NH-
   -NHCO-(CH₂)ₙ-X-(CH₂)ₚ
   -NHCO-(CH₂)ₙ-X-(CH₂)ₚ-NH-
   -NHSO₂-(CH₂)ₙ-X-(CH₂)ₚ-
   -NHSO₂-(CH₂)ₙ-X-(CH₂)ₚ-NH-
   -(CH₂)ₙ-X-(CH₂)p-
   -(CH₂)ₙ-X-(CH₂)ₚ-NH
   -CO-(CH₂)ₙ-X-(CH₂)ₚ-
   -CO-(CH₂)ₙ-X-(CH₂)ₚ-NH-
   dans lesquelles n est un nombre entier allant de 1 à 6, p est égal à 0 ou est un nombre entier de 1 à 6, X représente CH₂, O, S, NH, CO, HC=CH-, -(CH₂)ᵣZ, (CH₂)ᵣNHZ, 〉C=CH(CH₂)ᵣZ, 〉C=CH(CH₂)ᵣNHZ, 〉N(CH₂)ᵣZ, 〉N(CH₂)ᵣNHZ, 〉CH-O(CH₂)ᵣZ, 〉CH-O-(CH₂)ᵣNHZ, 〉HCS(CH₂)ᵣZ, 〉HCS(CH₂)ᵣNHZ avec r= 1 à 6 ;
- Z est un groupe protecteur utilisable en synthèse peptidique ou un radical dérivé d'une molécule de marquage choisie dans le groupe des molécules susceptibles d'être utilisées comme marqueurs et des précurseurs de ces molécules, à condition que Z ne représente un groupe protecteur que lorsque R² est un support comportant un bras espaceur de liaison.

Le dérivé de nucléoside mentionné ci-dessus comporte ainsi une molécule de marquage qui peut être de différents types. Ainsi, on peut utiliser une molécule de marquage détectable directement, par exemple des marqueurs fluorescents ou phosphorescents, des molécules antigéniques ou des dérivés ayant une forte affinité pour d'autres molécules.

A titre d'exemples, les molécules de marquage directement détectables peuvent être des molécules capables de s'associer à l'avidine ou la streptavidine, des molécules pouvant donner lieu à une réaction du type antigène-anticorps ou des molécules ayant des propriétés fluorescentes.ou luminescentes.

On peut aussi utiliser des molécules de marquage capables de recevoir un élément radioactif juste au moment de leur utilisation. En effet, lorsqu'on utilise des atomes radioactifs, il est difficile d'introduire ces éléments bien avant l'utilisation en raison de leur période et des réactions de radiolyse qu'ils induisent. Aussi, selon l'invention, on utilise dans ce cas une molécule de marquage susceptible d'être marquée ensuite par un élément radioactif par exemple par de l'iode radioactif.

On peut bien entendu utiliser dans le dérivé de nucléoside de l'invention d'autres molécules de marquage, en particulier celles utilisées habituellement pour la détection des antigènes, des anticorps, ou des haptènes en immunologie.

L'utilisation de dérivés de nucléosides de ce type comme synthons pour la synthèse d'oligonucléotides par des procédés classiques présente de nombreux avantages.

En effet, ces dérivés de nucléosides ont une stabilité et une réactivité comparables à celles des dérivés utilisés habituellement pour la synthèse automatique d'oligonucléotides car ils sont capables de subir sans altération toutes les opérations de cette synthèse et ils ont des réactivités très proches de celles des dérivés de nucléosides commerciaux utilisés actuellement.

Ainsi, contrairement à ce qui se faisait avec les procédés antérieurs, on peut préparer directement et rapidement une sonde marquée sans manipulations supplémentaires par rapport à la synthèse de l'oligonucléotide normal en utilisant un dérivé de nucléoside comportant déjà l'élément marqueur, ce qui permet de simplifier la mise en oeuvre et d'améliorer les rendements finaux.

En effet, il suffit de 6 minutes pour ajouter la molécule de marqueur et ceci peut être effectué automatiquement alors qu'avec les méthodes de l'art antérieur, un à deux jours sont nécessaires pour réaliser le marquage.

Le rendement est voisin de 100% pour chaque addition de dérivé portant un marqueur alors qu'il est beaucoup plus faible avec les autres méthodes où l'on effectue une réaction chimique sur l'oligonucléotide suivie d'étapes de purification.

On peut automatiser cette préparation de la sonde marquée comme dans le cas des synthèses d'oligonucléotides.

On peut introduire une ou plusieurs molécules de marquage de façon parfaitement définie quant au nombre et à la position dans la chaîne de l'oligonucléotide.

De plus, on peut utiliser des marqueurs différents sur certains sites particuliers de la chaîne oligonucléotidique, tout en obtenant un oligonucléotide de haute pureté.

Les dérivés de nucléosides de l'invention sont par ailleurs bien adaptés à l'utilisation d'atomes radioactifs car il est possible d'introduire ceux-ci juste avant l'utilisation de l'oligonucléotide, après avoir fixé sur celui-ci au cours de sa synthèse, une molécule capable d'être marquée facilement par cet élément radioactif.

A titre d'exemples, lorsque la molécule de marquage est une molécule capable de s'associer à l'avidine ou à la streptavidine, le radical Z peut répondre aux formules suivantes : 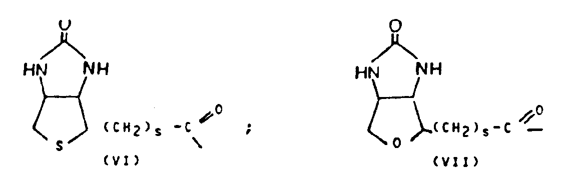 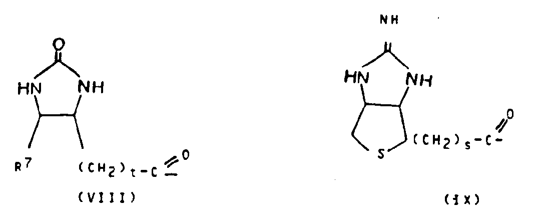 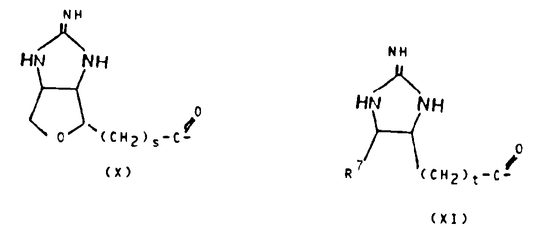
dans lesquelles
s=4, 5, 6 ou 7
t=3, 4, 5, 6 ou 7 et
R⁷ est un radical alkyle.

Parmi ces radicaux, le premier radical de formule VI avec s=4 est dérivé de la biotine et possède une forte affinité pour l'avidine.

A titre d'exemples, lorsque la molécule de marquage est une molécule pouvant donner lieu à une réaction du type antigéne-anticorps, le radical Z peut répondre aux formules suivantes :

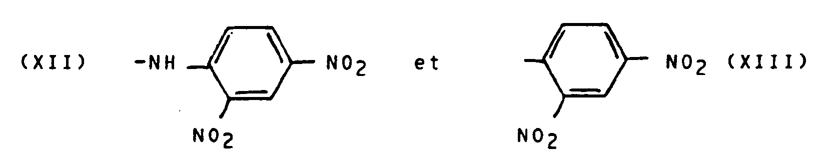

A titre d'exemples, lorsque la molécule de marquage est une molécule possèdant des propriétés fluorescentes, le radical Z peut répondre aux formules suivantes : 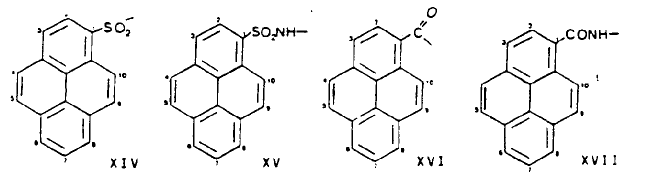 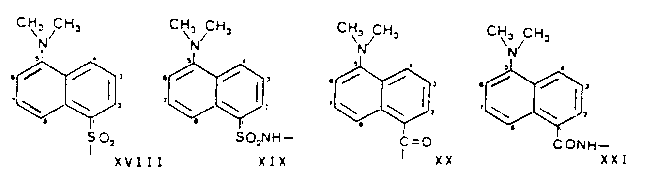 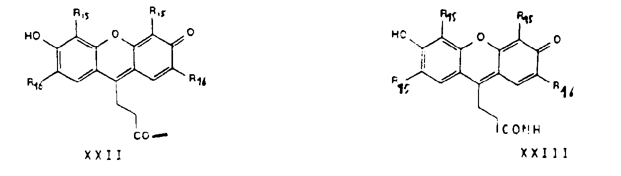
dans lesquelles R¹⁵ et R¹⁶ qui peuvent être identiques ou différents,sont un atome d'hydrogène ou un radical alkyle.

Lorsque la molécule de marquage est une molécule pouvant être marquée à l'iode, on utilise de préférence une molécule comportant une ou plusieurs fonctions phénoliques ou amine aromatique primaire, secondaire ou tertiaire. Dans ce cas, les radicaux Z susceptibles d'être utilisés, peuvent répondre aux formules suivantes : 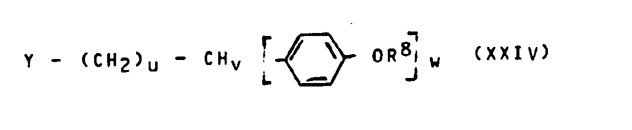
et 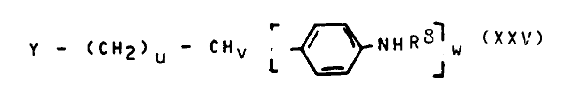
dans lesquelles u=0 ou est un nombre entier de 1 à 6,
v = 0,1 ou 2,
w = 3, 2 ou 1, avec v+w=3,
R⁸ représente un atome d'hydrogène ou un radical acyle, et
Y représente CH₂, NH, O, S, CO, SO₂, CONH ou SO₂NH.

Le radical acyle peut être par exemple le radical acétyle.

Selon l'invention, lorsque R² est un support comportant un bras espaceur de liaison, Z peut aussi représenter, un groupement protecteur de R⁴. Ce groupement protecteur peut être l'un des groupes protecteurs utilisés en synthèse peptidique, par exemple le groupement trifluoroacétyle. D'autres groupements utilisables tels que le Fmoc, sont décrits par Roduit et al dans Nucleosides & Nucleotides (1987), 6 n° 1 et 2, p. 349-352. Il peut s'agir aussi de groupements protecteurs utilisés en synthèse oligonucléotidique, lorsque R⁴ représente une simple liaison.

A titre d'exemple de tels radicaux protecteurs, on peut citer les radicaux benzoyle, anisoyle, isobutyryle ainsi que ceux décrits dans le brevet européen EP-A- 0 241 363.

De préférence, dans l'invention, on utilise un groupement benzoyle dans le cas des radicaux de formules (III) et (IV) et un groupement isobutyryle dans le cas du radical de formule (V).

Dans les dérivés de nucléosides de l'invention, le radical B dérivé d'une base purique ou pyrimidique peut comporter plusieurs groupements R⁴Z en position 2, 6 et/ou 8 pour les radicaux dérivés des bases puriques, soit dans le cas des radicaux de formules IV et V, ou en position 4, 5, 6 pour les radicaux dérivés des bases pyrimidiques, soit dans le cas des radicaux de formule III.

Les dérivés de nucléosides de l'invention sont destinés en particulier à être utilisés comme synthons pour la synthèse d'oligonucléotides marqués. Dans ce cas, le radical R¹ représente un radical labile en milieu acide convenant à la synthèse nucléotidique, et le radical R² représente un radical fonctionnel adapté à la synthèse nucléotidique. Selon que R³ représente un atome d'hydrogène ou un radical hydroxyle protégé ou non, on obtiendra un oligonucléotide du type ADN ou ARN.

A titre d'exemple de radicaux labiles en milieu acide susceptibles d'être utilisés pour former R¹, on peut citer en particulier les radicaux phényl-9-xanthényle et les radicaux trityle de formule : 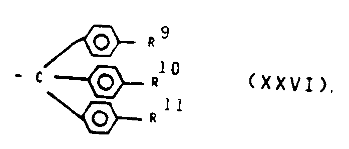
dans laquelle R⁹, R¹⁰ et R¹¹ qui peuvent être identiques ou différents, représentent un atome d'hydrogéne, un radical alkyle, alcoxy ou amino. De préférence, R¹ est le radical méthoxy ou diméthoxytrityle, soit le radical répondant à la formule XXIV précitée dans laquelle R⁹ ou R⁹ et R¹⁰ représentent des groupes méthoxy.

Pour la synthèse des oligonucléotides, le radical R² est un radical fonctionnel adapté à la synthèse oligonucléotidique, par exemple un radical phosphoré utilisable pour la synthèse phosphotriester, phosphodiester, phosphoramidite ou phosphonate d'oligonucléotides, ou un radical R¹²CO dans lequel R¹² représente une chaîne permettant l'accrochage sur un support par l'intermédiaire éventuellement d'une chaîne alkylaminée.

A titre d'exemple de radicaux phosphorés susceptibles d'être utilisés, on peut citer les radicaux de formule : 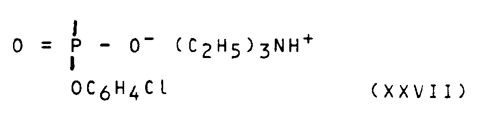 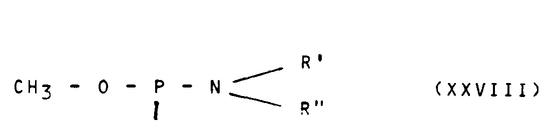 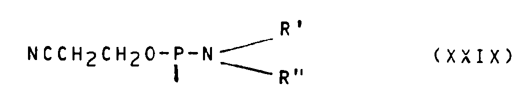 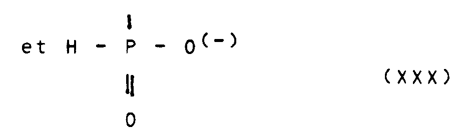
dans lesquelles R' et R'' pris séparément représentent chacun un radical alkyle, aralkyle ou cycloalkyle contenant jusqu'à 10 atomes de carbone, ou R' et R'' forment ensemble une chaîne alkylène contenant jusqu'à 5 atomes de carbone, ou forment ensemble avec l'atome d'azote un hétérocycle pouvant contenir un ou plusieurs autres hétéroatomes choisis parmi N, O, et S.

De préférence, lorsque R' et R'' sont pris séparément, ils représentent des groupes alkyle inférieur comme les radicaux isopropyle, t-butyle, isobutyle, sec-butyle, néopentyle, tert-pentyle, isopentyle, sec-pentyle.

De préférence, lorsque R' et R'' forment ensemble avec l'atome d'azote un hétérocycle, cet hétérocycle est la pyrrolidine, la morpholine ou la pipéridine.

A titre d'exemple, R² représente le radical de formule :

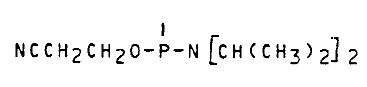

Pour la synthèse d'oligonucléotides, R² peut aussi être formé d'un support utilisable pour la synthèse d'oligonucléotides, comportant un bras espaceur de liaison avec l'atome d'oxygène.

Les supports peuvent être de différents types. Généralement, ils sont à base de silice et constitués par des grains de diamètre contrôlé, situé par exemple dans la gamme de 50 à 100nm. Le bras espaceur doit permettre la fixation du nucléoside sur le support. Il peut être formé par une chaîne alkylaminée comportant par exemple un radical dérivé d'un diacide tel que l'acide succinique, comme radical de liaison avec l'atome d'oxygène du nucléoside.

Lorsque R³ représente un radical hydroxyle protégé, on utilise comme groupe protecteur de cet hydroxyle, les groupements habituellement utilisés dans la synthèse des ribonucléotides.

Les dérivés de nucléosides répondant à la formule I dans laquelle R¹ est un radical protecteur et R² est un radical fonctionnel adapté à la synthèse nucléotidique peuvent être utilisés pour la préparation chimique directe d'oligonucléotides comportant au moins une molécule de marquage qui peut être, comme on l'a vu précédemment, une molécule détectable directement ou une molécule susceptible d'être transformée en molécule détectable par des méthodes classiques.

Cette préparation chimique de l'oligonucléotide peut être effectuée soit par la synthèse du phosphotriester lorsque R² est le radical de formule (XXVII), soit par la synthèse du phosphoramidite dans le cas où R² est le radical de formule (XXVIII) ou (XXIX), soit par la synthèse des phosphonates dans le cas où R² est le radical de formule (XXX).

La synthèse peut être effectuée soit par des méthodes en solution, soit par des méthodes de synthèse sur support. On utilise de préférence les méthodes de synthèse sur support qui donnent actuellement les meilleurs résultats.

Le procédé de synthèse d'oligonucléotides conforme à l'invention consiste à effectuer plusieurs cycles de condensation dans chacun desquels on condense un dérivé de nucléoside sur un dérivé de nucléoside ou sur un oligonucléotide, et il se caractérise en ce que l'un au moins des dérivés de nucléoside utilisé est un dérivé de nucléoside répondant à la formule : 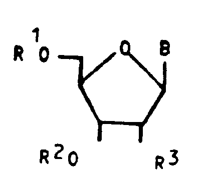
dans laquelle :
- R¹ représente un radical labile en milieu acide,
- R² représente un radical fonctionnel adapté à la synthèse nucléotidique ou un support utilisable pour la synthèse nucléotidique, comportant un bras espaceur de liaison avec l'atome d'oxygène,
- R³ représente un atome d'hydrogène, le radical hydroxyle ou un radical hydroxyle protégé, et
- B a la signification donnée ci-dessus.

Lorsque l'on utilise dans le procédé décrit ci-dessus plusieurs dérivés de nucléosides conformes à l'invention comportant des Z différents, on obtient un oligonucléotide marqué dans lequel différents nucléotides de la chaîne oligonucléotidique portent des marqueurs différents, ce qui n'était pas possible en utilisant les procédés de l'art antérieur.

Aussi, la présente invention a également pour objet un oligonucléotide marqué comportant une séquence de différents nucléotides, dans lequel au moins deux nucléotides de la séquence sont marqués par des marqueurs différents, ou dans lequel au moins deux nucléotides voisins situés à l'extrémité 5' de la séquence sont marqués par des molécules de marquage ou des précurseurs de ces molécules.

Les dérivés de nucléosides de l'invention peuvent ainsi trouver des applications pour la préparation des sondes froides marquées, par exemple par la biotine ou un de ses analogues, par un dérivé fluorescent tel que le dansyle ou le pyrène sulfonyle ou par un réactif antigénique tel que le dinitrophényle.

Ces dérivés de nucléosides peuvent aussi être utilisés pour la préparation de sondes pouvant ensuite être marquées spécifiquement à l'iode radioactif sur la ou les molécules de marquage Z utilisées dans les dérivés de nucléosides de départ.

Les dérivés de nucléosides de l'invention peuvent aussi être utilisés pour la synthèse d'amorces, par exemple dans les méthodes d'amplification du type PCR.

L'invention a encore pour objet des oligonucléotides marqués comportant une séquence de différents nucléotides dans laquelle au moins l'un des nucléotides est marqué par un radical choisi parmi
- les radicaux de formule 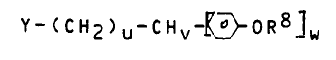 ou 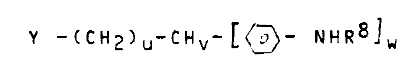 dans lesquelles
   u = 0 ou est un nombre entier de 1 à 6,
   v = 0, 1 ou 2,
   w = 3, 2 ou 1 avec v+w=3,
   R⁸ représente un atome d'hydrogène ou un radical acyle, et
   Y représente CH₂, NH, O, S, CO, SO₂, CONH, ou SO₂NH,
- les radicaux de formule : 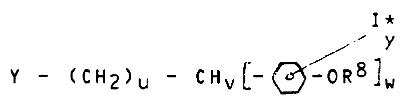 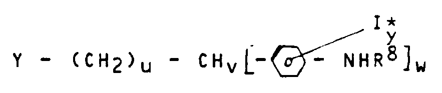 dans lesquelles R⁸ est un atome d'hydrogène et u, v, w, et Y ont la signification donnée ci-dessus, y=1, 2, 3 ou 4 et I* est un atome d'iode radioactif ;
- les radicaux de formule : 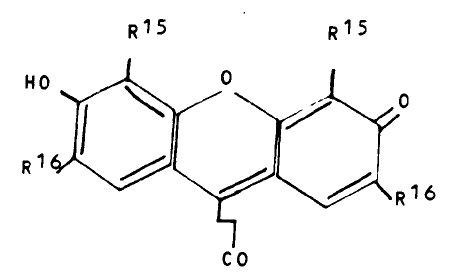 dans laquelle R¹⁵ et R¹⁶ qui peuvent être identiques ou différents, représentent un atome d'hydrogène ou un radical alkyle ; et
- les radicaux pyrényle de formule :

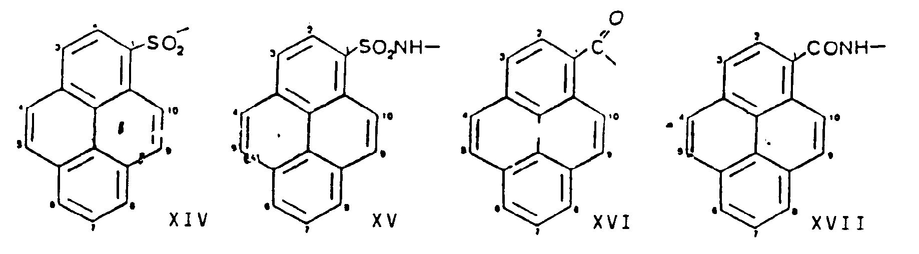

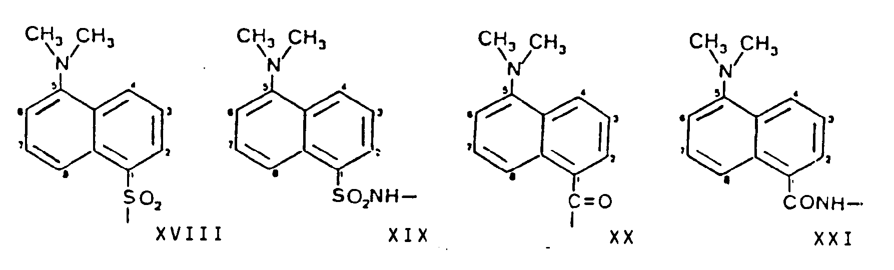

L'invention concerne également un oligonucléotide marqué comportant une séquence de différents nucléotides, caractérisé en ce qu'au moins 5 nucléotides situés à au moins l'une des extrémités de la séquence sont marqués par des molécules de marquage ou des précurseurs de ces molécules.

Ce mode de réalisation de l'oligonucléotide est très intéressant car la présence d'au moins 5 molécules de marquage à une extrémité de la séquence permet d'améliorer la sensibilité.

Les dérivés de nucléosides de l'invention peuvent être préparés par des procédés classiques tels que ceux décrits dans Organic Chemistry of Nucleic Acids. Part B.N.K. Kochetkov and E.I. Budovski, 1972 (Plenum New York). Généralement, on part des dérivés de nucléosides répondant à la formule : 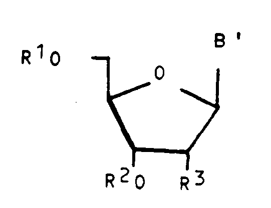
dans laquelle R¹, R², R³ ont les significations données ci-dessus, et B' représente un radical de formule : 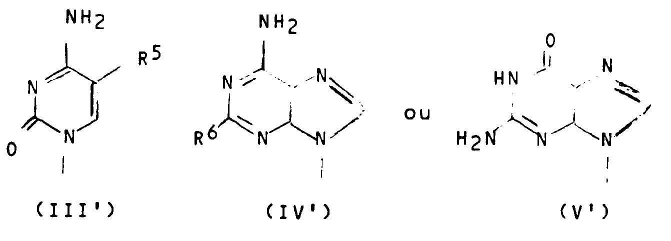
avec R⁵ représentant H ou CH₃, et on fixe sur ceux-ci un ou plusieurs radicaux R⁴Z par des méthodes classiques.

Lorsque le radical B du dérivé de nucléoside à préparer est un radical de formule (III), on peut aussi préparer ce dérivé en partant de la 4-thiothymidine ou de la 4-thiodésoxyuridine protégée ou non, par réaction avec une diamine, un dérivé monosubstitué de diamine ou une molécule de marquage H₂N-R⁴Z dans le cas où celle-ci peut être couplée à une fonction amine primaire. Ceci correspond au schéma réactionnel suivant : 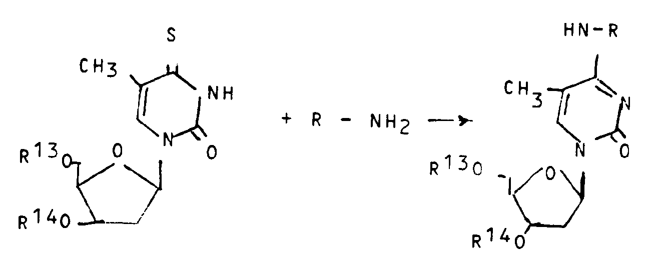
dans lequel R¹³ et/ou R¹⁴, qui peuvent être identiques ou différents, représentent H ou un radical protecteur, et R est un radical hydrocarboné, par exemple R⁴Z.

Après cette réaction, on peut traiter le dérivé de nucléoside pour le rendre apte à la synthèse nucléotidique et lui ajouter une ou plusieurs molécules de marquage si nécessaire, en particulier lorsque R n'est pas le groupe R⁴Z.

D'autres caractéristiques et avantages de l'invention apparaîtront mieux à la lecture des exemples suivants donnés bien entendu à titre illustratif et non limitatif.

### Exemple 1 : Préparation du composé n° 1 qui est le dérivé de nucléoside de formule :

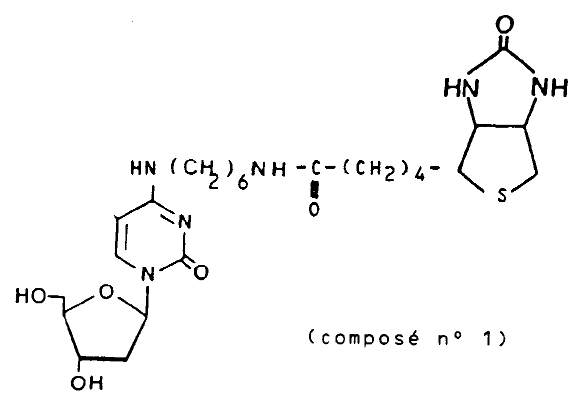

Dans ce dérivé de nucléoside de formule (I), R¹, R² et R³ représentent un atome d'hydrogène, B représente le radical de formule (III), R⁵ représente un atome d'hydrogène, R⁶ représente le radical R⁴Z, R⁴ représente -(CH₂)₆NH-, Z est un radical dérivé de la biotine et m=0.

### a) Préparation du composé n° 2 de formule :

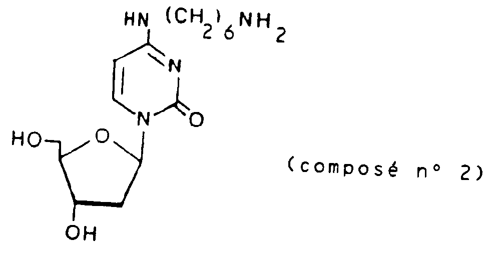

On dissout 0,26g (1mmol) de 4-thiodésoxyuridine dans 5ml d'éthanol absolu, et on y ajoute 0,58g (5mmol) de 1,6-diaminohexane, et on maintient pendant 16h à 60°C. On évapore à sec, reprend par 10ml d'eau et 0,2ml de triéthylamine et on évapore. On triture le résidu par 4x10ml d'éther éthylique. On adsorbe le résidu sur 1g de Célite 545 et on le dépose sur une colonne de Célite préparée avec la phase inférieure du mélange acétate d'éthyle-méthoxyéthanol-eau 4 : 1 : 2, on élue la colonne par la phase supérieure de ce mélange puis on élue le produit recherché par la phase supérieure du mélange : acétate d'éthyle n-butanol-eau 1 : 1 :1. Par évaporation des fractions appropriées et lyophilisation, on détient 220mg (65%) du composé n° 2.

### b) Préparation du composé n° 1.

On dissout 653mg (2mmol) du composé n° 2 dans 15ml de diméthylformamide et on ajoute 0,4ml (4mmol) de triéthylamine et 700mg (2mmol) de N-hydroxysuccinimido biotine. Après 3h sous agitation à 20°C, on évapore sous vide le mélange réactionnel, puis on le coévapore avec 2 fois 15ml d'eau distillée puis avec 2 fois 20ml d'éthanol absolu. On reprend le résidu par du méthanol, on ajoute 8g de gel de silice (0,2 à 0,5mm) et on sèche la suspension sous vide. On dépose alors sur une colonne de gel de silice (gel 60 de Merck) le mélange adsorbé préalablement sur la silice, et on élue ensuite la colonne par un gradient de méthanol dans le chloroforme (5 à 20%). On obtient ainsi 740mg du composé n°1, ce qui correspond à un rendement de 65%.

### Exemple 2 : Préparation du composé n° 3, qui est le dérivé de nucléoside de formule :

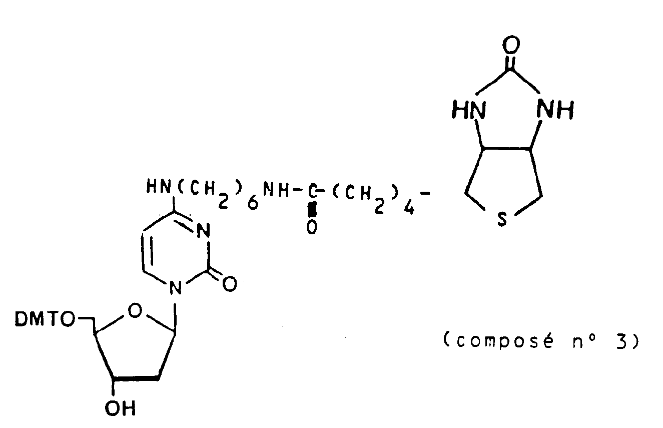

On dissout à chaud 1,11g (2mmol) du composé n° 1 dans de la pyridine anhydre, on refroidit la solution et on l'évapore sous vide. On reprend le résidu par 5ml de diméthylformamide anhydre et on dilue par 20ml de pyridine anhydre, puis on refroidit le mélange à 0°C sous agitation. On ajoute alors 744mg de chlorure de diméthoxy-4,4'-trityle. On maintient le mélange pendant 16h sous agitation. On ajoute alors 2ml de méthanol, puis après 15min on verse le mélange réactionnel sur 35ml d'une solution aqueuse saturée de bicarbonate de sodium.

On extrait le mélange par deux fois 50ml de chloroforme, puis on évapore la phase organique et on la coévapore avec deux fois 20ml de toluène.

On reprend le résidu par du dichlorométhane et on le purifie par chromatographie sur gel de silice (gel 60 de Merck) en utilisant pour l'élution un gradient de méthanol dans du dichlorométhane. On obtient ainsi 1,38g du composé n°3, ce qui correspond à un rendement de 81%.

### Exemple 3 : Préparation du composé n° 3.

Dans cet exemple, on prépare le composé n° 3 à partir du composé n° 4 de formule :

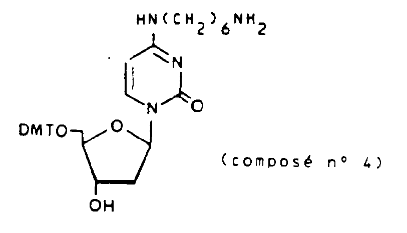

### a) Préparation du composé n° 4.

On dissout 3,7g (6,8mmol) de diméthoxytrityle-5'-thio-4-désoxy-2'-uridine dans 40ml d'éthanol absolu, et on ajoute 3,9g (34mmol) de 1,6-diaminohexane et on maintient pendant 16h à 60°C. Après évaporation et partition entre chloroforme et soude 0,1N, la phase organique est lavée par l'eau jusqu'à neutralité puis évaporée à sec.

Le résidu est repris par du dichlorométhane et chromatographié sur gel de silice G Merck en utilisant pour l'élution un gradient de méthanol dans le chloroforme.

On obtient 3,65g de composé n°4, ce qui correspond à un rendement de 85%.

### b) Préparation du composé n° 3.

On dissout 3,15g (5mmol) du composé n° 4 dans 50ml de diméthylformamide. On ajoute à cette solution, 1,7g (5mmol) de N-hydroxysuccinimido biotine et 770µl (5,5mmol) de triéthylamine. Après 3h sous agitation à la température ambiante, on évapore le solvant et on reprend le résidu par 500ml de chloroforme. On lave deux fois la solution chloroformique par 500ml de bicarbonate de sodium à 10%, puis par 500ml d'eau distillée. On évapore la phase organique à sec. On reprend le résidu par du dichlorométhane et on le soumet à une chromatographie sur gel de silice 60 (Merck) en réalisant l'élution par un gradient de méthanol dans le chloroforme. On évapore les fractions contenant le produit recherché. On obtient ainsi 3,2g du composé n° 3, ce qui correspond à un rendement de 75%.

### Exemple 4 : Préparation du composé n° 5 de formule :

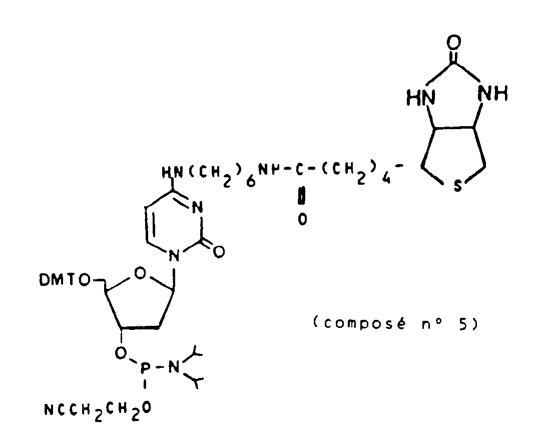

On rend anhydre 854mg (1mmol) du composé n° 3 par coévaporation dans la pyridine absolue, puis on redissout dans 10ml de dichlorométhane anhydre. On ajoute 85mg (0,5mmol) de tétrazolate de diisopropylammonium ainsi que 341µl de bis(diisopropylamino)cyanoéthoxyphosphine. On agite le milieu réactionnel pendant 2h à la température ambiante, puis on ajoute 50ml de dichlorométhane. On lave la solution organique par trois fois 50ml de bicarbonate de sodium en solution aqueuse saturée, puis par 50ml d'une solution saturée de chlorure de sodium.

On évapore sous vide la phase organique, on reprend le résidu par 5ml de dichlorométhane et on le précipite dans 100ml d'hexane refroidi à -78°C. On collecte le précipité et on sèche sous vide. On obtient ainsi 897mg du composé n° 5, ce qui correspond à un rendement de 85%.

### Exemple 5 : Préparation du composé n° 6 de formule :

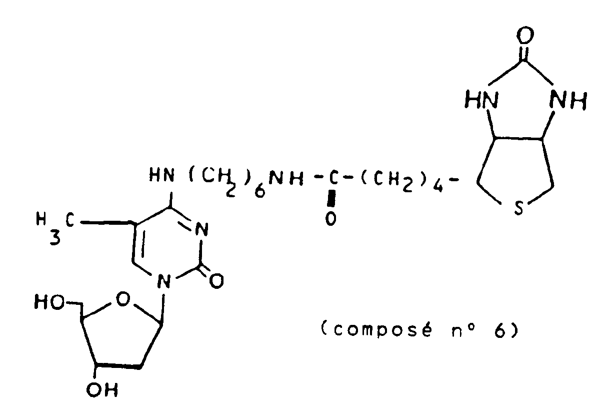

### a) Préparation du composé n° 7 de formule :

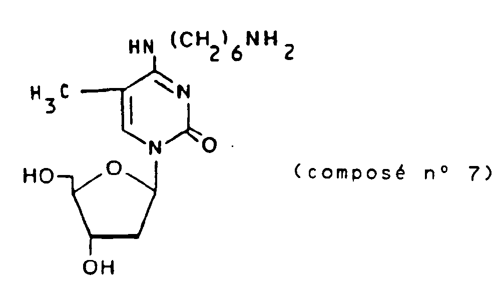

On suit le même mode opératoire que dans l'exemple 1a) pour préparer le composé n° 7 en utilisant la 4-thiothymidine au lieu de la 4-thiodésoxyuridine. On obtient ainsi le composé n° 7.

### b) Préparation du composé n° 6.

On suit le même mode opératoire que dans l'exemple 1b) pour préparer le composé n° 6 à partir du composé n° 7 en utilisant 681mg (2mmol) du composé n° 7, 15ml de diméthylformamide, 0,4ml (4mmol) de triéthylamine et 700mg (2mmol) de N-hydroxysuccinimidobiotine.

On obtient ainsi 715mg du composé n° 6, ce qui correspond à un rendement de 63%.

### Exemple 6 : Préparation du composé n° 8 de formule :

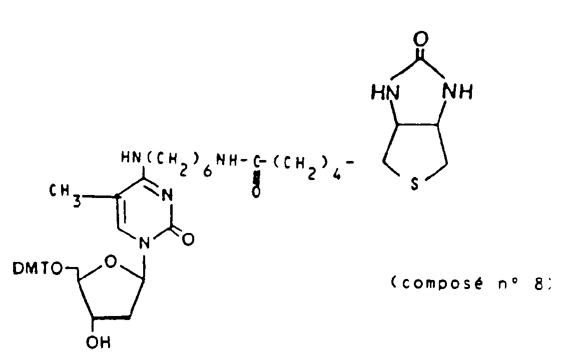

On prépare ce composé à partir du composé n° 6 en suivant le même mode opératoire que dans l'exemple 2 et en utilisant 1,14g (2mmol) du composé n° 6 et 744mg de chlorure de diméthoxy-4,4'-trityle.

On obtient ainsi 1,44g du composé n° 8, ce qui correspond à un rendement de 83%.

### Exemple 7 : Préparation du composé n° 8 à partir du composé n° 9 de formule :

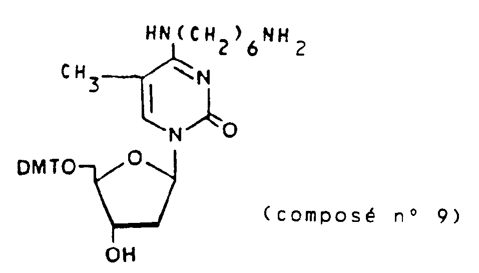

### a) Préparation du composé n° 9.

On suit le même mode opératoire que dans l'exemple 3a) pour préparer le composé n° 9 en utilisant la diméthoxytrityle-5'-thio-4-thiothymidine au lieu de la diméthoxytrityle-5'-thio-4-désoxy-2'uridine.

### b) Préparation du composé n° 8.

On suit le même mode opératoire que dans l'exemple 3 pour préparer le composé n° 8 à partir du composé n° 9 en utilisant 1,29g (2mmol) du composé n° 8, 20ml de diméthylformamide, 680mg de N-hydroxysuccinimidobiotine et 300µl (2mmol) de triéthylamine. Après 3h de réaction, on obtient 1,37g du composé n° 8, ce qui correspond à un rendement de 79%.

### Exemple 8 : Préparation du composé n° 10 de formule :

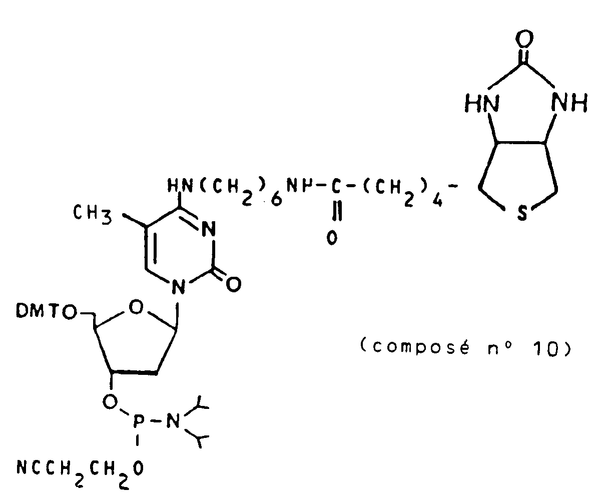

On suit le même mode opératoire que dans l'exemple 4 pour préparer le composé n° 10 à partir du composé n° 8 en utilisant 870mg (1mmol) du composé n° 8, 85mg de tétrazolate de diisopropylammonium et 340µl de bis(diisopropylamino)cyanoéthoxyphosphine.

Après la précipitation finale, on obtient 930mg du composé n° 10, ce qui correspond à un rendement de 87%.

### Exemple 9 : Synthèse d'un oligonucléotide marqué à la biotine.

Cet exemple illustre l'utilisation du composé n° 5 pour préparer un oligonucléotide marqué à la biotine dont la séquence est la suivante :

5'-d(XGTCATAGCTGTTTCCTG)-3'

Dans cette séquence, A représente le nucléotide formé par l'adénine, C représente le nucléotide formé par la cytosine, G représente le nucléotide formé par la guanine, T représente le nucléotide formé avec la thymine et X représente le nucléotide formé avec la (biotinamido-6-hexyl-1)-4-cytosine.

Pour effectuer cette synthèse, on utilise le composé n°5 comme synthon correspondant à la (biotinamido-6-hexyl-1)-4-cytosine et des synthons correspondant à l'adénosine, à la cytosine, à la guanine et à la thymine. Ceux-ci sont obtenus à partir des nucléosides correspondants en protégeant la fonction hydroxyle en 5' par le radical diméthoxy-4,4' trityle et en fonctionnalisant la fonction hydroxyle en 3' par le radical de formule :

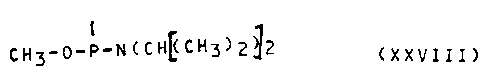

Pour les nucléotides correspondant à l'adénine et à la cytosine, la fonction aminée exocyclique est protégée par le groupement benzoyle. Pour la guanine, la fonction aminée exocyclique est protégée par le groupement isobutyryle.

La synthèse a été effectuée au moyen d'un synthétiseur automatique d'oligonucléotides Applied Biosystems 381 A en utilisant :
- 0,2µmoles de nucléoside greffé sur un support "Controlled Pore Glass" comportant un bras espaceur formé par une chaîne aminée, la liaison entre ce nucléoside et l'amine étant réalisée par un radical succinyle.
- 10mg par cycle de condensation du dérivé de nucléoside (composé n° 5) obtenu dans l'exemple 4 ou des synthons de désoxy -2'-adénosine, de désoxy-2'-cytidine, de désoxy-2'-guanosine et de thymidine, soit environ 40 équivalents par rapport au support.
- 7mg par cycle de condensation d'un agent d'activation constitué par du tétrazole, soit environ 10 équivalents par rapport au nucléotide.

Le cycle de condensation est le cycle standard "0,2µmoles, cyanoéthylphosphoramidites" de l'appareil Applied Biosystems 381 A.

A la fin du cycle de condensation, on obtient l'oligonucléotide protégé marqué à la biotine lié de façon covalente au support par l'intermédiaire du bras espaceur succinylé.

L'oligonucléotide est coupé du support par traitement à l'ammoniaque à 28% pendant 2 heures puis la solution ammoniacale obtenue est chauffée 5 à 16 heures à 60°C pour éliminer les groupements protecteurs. L'ammoniaque est évaporée. Le résidu est repris par 500µl d'eau distillée et filtré sur une colonne de Sephadex G 25 (diamètre 1cm, hauteur 7cm). Les fractions correspondant au premier pic d'absorption à 254mm sont recueillies et lyophilisées.

On vérifie la bonne longueur de l'oligonucléotide biotinylé synthétisé par marquage au phosphore 32, par rapport à des témoins, au moyen de la polynucléotide kinase, et par électrophorèse sur gel de polyacrylamide.

Le produit est préparé par électrophorèse sur gel de polyacrylamide épais. La bande correspondant au composé est découpée (après visualisation en ultraviolet).

L'oligonucléotide obtenu après élution, essayé en parallèle avec un oligonucléotide comportant une séquence identique mais dont le marquage avec la biotine a été fait suivant les méthodes conventionnelles a montré la même sensibilité pour la détection d'une séquence complémentaire.

### Exemple 10 : Synthèse d'un oligonucléotide marqué à la biotine.

On prépare de la même façon que dans l'exemple 9 un oligonucléotide marqué à la biotine dont la séquence est la suivante :

5'-d (YGTCATAGCTGTTTCCTG)- 3'

Dans cette séquence, Y représente le nucléotide formé avec la (biotinamido-6-aminohexyl-1)-4-méthyl-5-cytosine ; le synthon correspondant utilisé pour Y est le composé n° 10.

On vérifie de la même façon que dans l'exemple 9 la bonne longueur de l'oligonucléotide biotinylé synthétisé ainsi que sa sensibilité pour la détection d'une séquence complémentaire.

### Exemple 11 : Synthèse d'un oligonucléotide marqué à la biotine.

On suit le même mode opératoire que dans l'exemple 9 pour préparer l'oligonucléotide marqué à la biotine dont la séquence est la suivante :

5'-d (XXXGTCATAGCTGTTTCCTG)-3'

Dans cette séquence, X représente, comme dans l'exemple 9, le nucléotide formé avec la (biotinamido-6-hexyl-1)-4-cytosine ; le synthon correspondant est le composé n° 5.

On vérifie de la même façon que dans l'exemple 9 la bonne longueur de l'oligonucléotide biotinylé synthétisé ainsi que sa sensibilité.

Dans le cas de cet oligonucléotide qui comporte trois nucléotides biotinylés, la réponse est proportionnelle au nombre de biotines puisque la sensibilité est multipliée par un facteur voisin de 3.

On prépare de la même façon un oligonucléotide marqué à la biotine dont la séquence est la suivante : 
5'-d(XXXX XXX XXX TTT CCT GTT GCT GTG AGC TTG CTG
AGC TAA CTG CTC TGC CCT GGC)-3'
en utilisant les mêmes synthons.

On obtient ainsi un oligonucléotide comportant 10 nucléotides biotinylés à l'extrémité 5' de sa séquence.

On prépare de la même façon un oligonucléotide marqué à la biotine dont la séquence est la suivante :

5'-d(XXXXX TTT CCT GTT GCT GTG AGC TTG CTG AGC TAA
CTG      CTC TGC CCT GGC)-3'

On obtient ainsi un oligonucléotide comportant 5 nucléotides biotinylés à l'extrêmité 5' de la séquence.

### Exemple 12 : Préparation du composé n° 11 de formule :

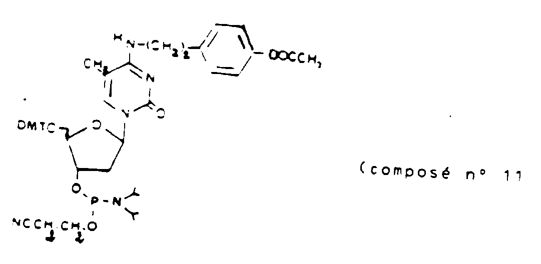

dans laquelle DMTO représente le radical diméthoxytrityle.

Pour ce dérivé de nucléoside de formule 1, où B est le radical de formule (III) dérivé de la cytosine, on effectue la préparation en partant de la thiothymidine (composé n° 12) par la succession d'étapes suivantes a) à f).

### a) Préparation du dérivé monométhoxytritylé de la thiothymidine (composé n° 13) à partir de la thiothymidine (composé n° 12).

Cette réaction correspond au schéma réactionnel suivant : 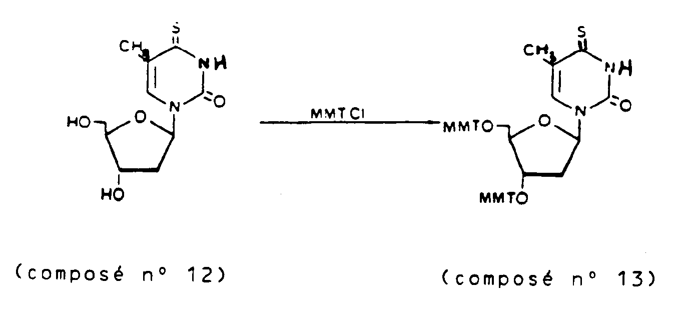
dans lequel MMTCl représente le chlorure de monométhoxytrityle et MMT représente le radical monométhoxytrityle.

Cette réaction a pour but de protéger les hydroxyles osidiques en 5' et en 3' afin d'éviter leur acétylation lors de l'introduction du groupement R⁴Z qui est le groupe 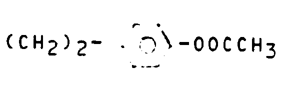
dans le cas du composé n° 11.

On fait réagir 5mmol de thiothymidine après séchage à la pompe à palettes avec 15mmol de chlorure de monométhoxy trityle (MMTCl) dans 50ml de pyridine anhydre. On laisse la réaction se poursuivre pendant 16 heures à la température ambiante, puis pendant 5 heures à 50°C. On arrête alors la réaction par 5ml de méthanol et on reprend le mélange réactionnel par 250ml de chloroforme. On lave la solution obtenue 3 fois par 250ml de NaHCO₃ à 5%, puis par 250ml d'eau distillée.

On sépare le produit obtenu sur colonne de silice en utilisant pour l'élution un gradient CH₂Cl₂/hexane avec un rapport 9/1 jusqu'à CH₂Cl₂ pur. On précipite le produit à l'hexane pour éliminer le tritanol. On obtient ainsi le composé n° 13 avec un rendement de 87%.

### b) Préparation du composé n° 14 de formule :

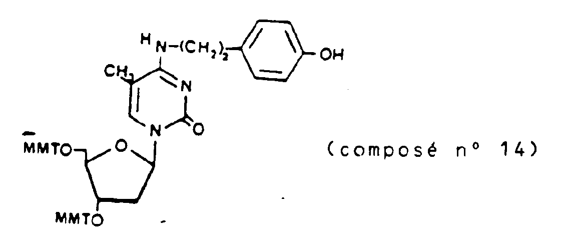

par réaction du composé n° 13 avec la tyramine de formule :

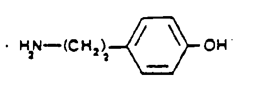

On fait réagir 5mmol du composé n° 13 et 20mmol de tyramine (4éq.) pendant 24 heures à 60°C en solution dans 10ml d'éthanol dans un flacon de 25ml. On évapore le mélange à sec et on reprend par 100ml de chloroforme, puis on lave 3 fois par 100ml d'eau distillée. On sépare le produit obtenu sur colonne de silice dans un gradient chloroforme-chloroforme/méthanol (93/7).

### c) Préparation du composé n° 15 de formule :

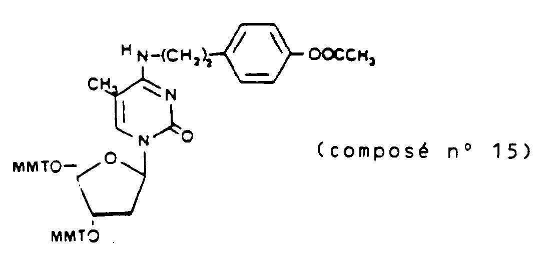

par réaction du composé n° 14 avec l'anhydride acétique.

On sèche par coévaporation dans la pyridine anhydre 3mmol du composé n° 14 et on les fait réagir pendant 4 heures dans 30 ml de pyridine anhydre avec 3ml d'anhydride acétique (environ 10 éq.). On arrête la réaction par 3ml de méthanol et on évapore à sec. Après extraction par un mélange chloroforme-bicarbonate de sodium, on évapore à sec la phase organique et on la sépare sur silice. On élue le produit par CH₂Cl₂-méthanol : 98/2. Le rendement est de 82%.

### d) Préparation du composé n° 16 de formule :

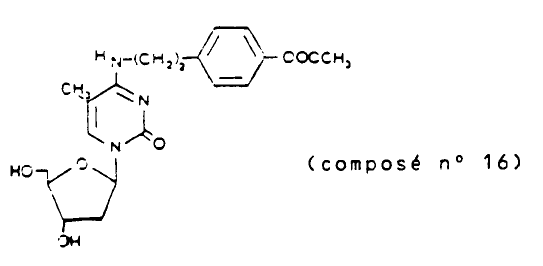

par réaction du composé n° 15 avec l'acide acétique.

On fait réagir 3mmol du composé n° 15 avec 30ml d'acide acétique à 80% au reflux, pendant une demi-heure, puis on évapore à sec. On reprend le résidu par de l'eau distillée, puis on évapore l'eau, et on reprend le résidu par 50ml d'eau distillée. On lave la phase aqueuse deux fois par 50ml de toluène, puis on l'évapore à sec. On obtient ainsi le composé n° 16. Le rendement n'est pas mesuré car ce composé est utilisé sans séparation.

### e) Préparation du composé n° 17 de formule :

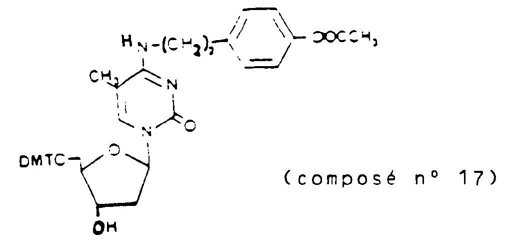

On sèche par coévaporation dans la pyridine anhydre le composé n° 16 obtenu précédemment, puis on le trityle en 5' par 1,2 équivalent de chlorure de p-diméthoxytrityle dans 30ml de pyridine anhydre à 0°C.

On reprend le mélange réactionnel par 200ml de chloroforme, puis on lave la solution organique obtenue trois fois par 200ml de NaHCO₃ à 5%, puis par 100ml d'eau distillée. On chromatographie le produit obtenu sur colonne de silice et on recueille le composé n° 17. Le rendement est de 69% pour les 2 étapes.

### f) Préparation du composé n° 11 à partir du composé n° 17.

On sèche par coévaporation dans l'acétonitrile anhydre le composé n° 17 obtenu précédemment.

On dissout 0,5mmol du composé n° 17 dans 5ml de CH₂Cl₂ et on ajoute 0,25mmol de tétrazolate de diisopropylammonium et 1,1mmol de cyanoéthoxy, bis diisopropylaminophosphine. On laisse la réaction se poursuivre pendant 2 heures, puis on ajoute 50ml de dichlorométhane. On lave la solution organique par 3 fois 50ml de NaHCO₃ à 5%, puis par 50ml d'eau distillée et on évapore après séchage sur sulfate de sodium. On reprend le résidu par 5ml de dichlorométhane et on précipite le composé n° 11 dans 100ml d'hexane à -80°C. On place ensuite le précipité pendant au moins une journée dans un dessicateur sous vide.

### Exemple 13 : Préparation d'un oligonucléotide marqué.

On suit le même mode opératoire que dans l'exemple 9 pour préparer un oligonucléotide marqué à l'iode dont la séquence est la suivante :

5'-d(XGTCATAGCTGTTTCCTG)-3'

Dans cette séquence A, C, G, T, représentent les mêmes nucléotides que dans l'exemple 9 et X représente le nucléotide formé avec l'((hydroxy-4 phényl)-2 éthyl)-N₄, méthyl-5 cytosine.

Pour cette synthèse, on utilise les mêmes synthons que dans l'exemple 9 pour A, C, G et T et le composé n° 11 comme synthon du nucléotide X.

On effectue la synthèse au moyen d'un synthétiseur automatique d'oligonucléotides Applied Biosystems 381A dans les mêmes conditions que celles de l'exemple 9.

A la fin du cycle de condensation, on obtient l'oligonucléotide protégé lié au support. On sépare l'oligonucléotide du support, on le déprotège et on le sépare dans des conditions similaires à celles de l'exemple 9.

On réalise ensuite le marquage par l'iode radioactif. Dans ce but, on ajoute à 1µmol de ¹²⁵I dans 9µl de tampon phosphate (0,05M, pH=7,5), 20pmol de l'oligonucléotide obtenu dans 10µl d'eau distillée et 10µl d'une solution de chloramine T(0,1nmol). Après 5 minutes, on arrête la réaction par du métabisulfite de sodium.

On fait subir à un oligonucléotide non marqué obtenu dans les mêmes conditions que précédemment, mais ne comportant pas le nucléotide X, le même traitement de marquage à l'iode. Dans ces conditions, on n'obtient aucun marquage de l'oligonucléotide, ce qui montre que la présence du groupe 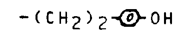
est essentielle pour le marquage à l'iode.

On vérifie comme dans l'exemple 9 que l'oligonucléotide marqué peut être utilisé pour la détection d'une séquence complémentaire par hybridation.

### Exemple 14 : Préparation du composé n° 18 de formule :

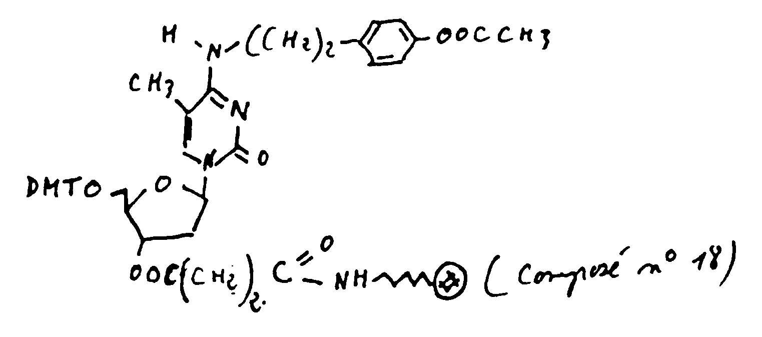

dans laquelle NH 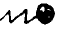 représente un support de silice ayant un bras espaceur aminé.

Pour ce dérivé de nucléoside de formule (I), où B est le radical de formule (III) dérivé de la cytosine, on effectue la préparation en partant du composé n° 17 par la succession d'étapes suivantes a) à c).

### a) Préparation du dérivé succinylé (composé n° 19) à partir du composé n° 17.

Au composé n° 17 (0,5 mmol - 353mg) séché par coévaporation dans la pyridine anhydre, sont ajoutés 10ml de pyridine anhydre, 1,5mmol de diméthylaminopyridine (DMAP) et 1,5mmol d'anhydride succinique. Au bout de 16 heures, la réaction est terminée. Le mélange réactionnel est évaporé à sec, repris par 100ml de chloroforme. La solution organique obtenue est lavée par 3 fois 100ml d'acide citrique. La phase organique est séchée sur Na₂SO₄ et évaporée à sec. Le résidu est repris par 10ml de chloroforme et précipité dans 200ml d'hexane. Le rendement est de 87%.

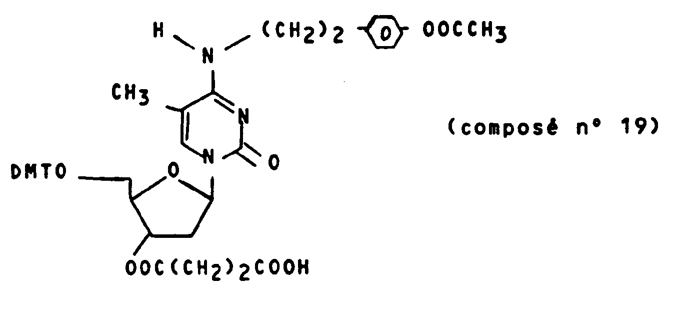

### b) Préparation de l'ester activé (composé n° 20) à partir du composé n° 19.

0,25mmol de composé n° 19 préalablement séché par coévaporation dans la pyridine anhydre, 0,28mmol de pentachlorophénol et 0,37mmol de dicyclohexyl carbodiimide (DCC) sont mis en réaction 16h dans 10ml de diméthylformamide anhydre.

Le mélange réactionnel est filtré. Le filtrat est évaporé à sec, repris par 100ml de chloroforme. La solution organique obtenue est lavée par 3 fois 100ml d'eau distillée, puis reprise par 5 à 10ml de chloroforme et précipitée dans 200ml d'hexane. Le rendement est de 93%.

### c) Préparation du composé n° 18 à partir du composé n° 20.

0,2mmol de composé n° 20 sont placés sous agitation mécanique avec 200mg de silice CPG ayant un bras espaceur alkylaminé dans 20ml de DMF anhydre avec 30µl de triéthylamine pendant 2 jours. Au bout de ce laps de temps le support greffé est récupéré et lavé par 3x20ml de DMF anhydre puis 3x20ml d'éthanol. Les fonctions aminées n'ayant pas réagi sont bloquées par acétylation au moyen de 5ml d'anhydride acétique dans 50ml de pyridine. Le support est récupéré et lavé par 3 x 20ml de pyridine et 3 x 20ml d'éthanol.

Le greffage du support a été mesuré par l'intensité de la coloration du cation diméthoxytrityle libéré en milieu acide. Support greffé à 27µmol/g.

On prépare de la même façon le composé n° 21 de formule 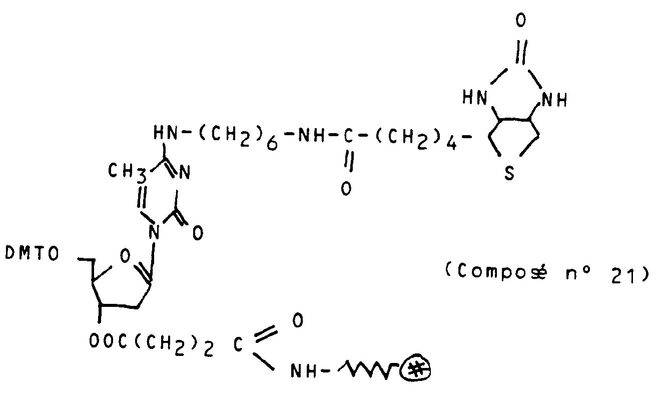
en partant du composé n° 8.

### Exemple 15 : Préparation du composé n° 22 de formule :

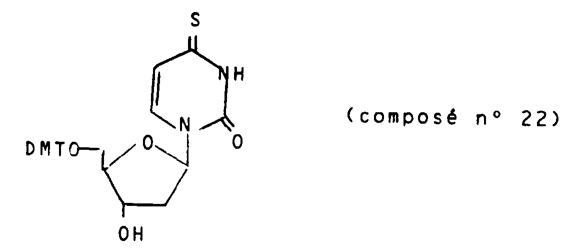

On sèche par coévaporation avec 2x10ml de pyridine anhydre, 4mmol de 4-thiodésoxy-2'-uridine. Le résidu est dissous dans 40ml de pyridine et la solution est refroidie dans un bain de glace. On ajoute alors 4,4mmoles (0,93g) de chlorure de diméthoxytrityle et la réaction est laissée sous agitation à 4°C pendant une nuit. Quand la réaction est jugée complète (CCM sur silice) 1ml de méthanol est ajouté et, après 30min, le mélange réactionnel est concentré au quart de son volume. Le résidu est repris dans 100ml de dichlorométhane, lavé par 3x100ml de bicarbonate saturé puis par 100ml d'eau. La phase organique est séchée sur sulfate de sodium, évaporée à sec puis coévaporée avec du toluène. Le produit est purifié sur colonne de gel de silice avec un gradient de méthanol dans le dichlorométhane (0 à 4%). On obtient le composé n°22 amorphe, jaune avec un rendement de 1,84g (84%).

Le composé n°22 peut être utilisé pour la préparation du composé n°3 de l'exemple 2, en suivant le même mode opératoire que dans l'exemple 1 et en utilisant ce composé à la place de la 4-thiodésoxyuridine.

### Exemple 16 : Préparation du composé n° 23 de formule :

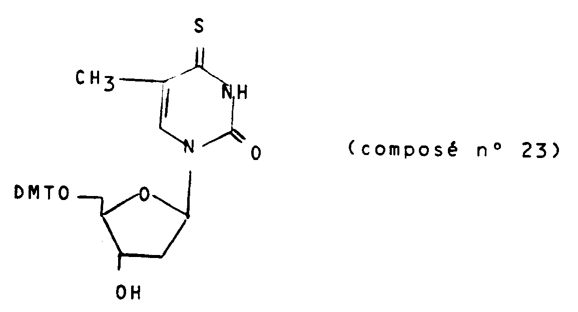

On suit le même mode opératoire que dans l'exemple 15 pour préparer le composé n° 23 à partir de 0,64g (2,5mmol) de thio-4-thymidine. On obtient ainsi 1,20g du composé n° 23, soit un rendement de 86%.

Le composé n° 23 peut être utilisé pour la préparation du composé n° 8 de l'exemple 6 en suivant le mode opératoire de l'exemple 1, mais en remplaçant la 4-thiodésoxyuridine par le composé n° 23.

## Revendications

1. Dérivé de nucléoside utilisable pour la synthèse d'oligonucléotides marqués, caractérisé en ce qu'il répond à la formule : 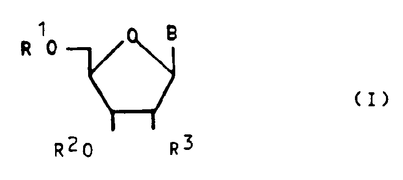 dans laquelle :
- R¹ représente un atome d'hydrogène, ou un radical labile en milieu acide convenant à la synthèse nucléotidique,
- R² représente un atome d'hydrogène, un radical protecteur, un radical fonctionnel adapté à la synthèse oligonucléotidique, ou un support utilisable pour la synthèse oligonucléotidique comportant un bras espaceur de liaison avec l'atome d'oxygéne,
- R³ représente un atome d'hydrogène, le radical hydroxyle ou un radical hydroxyle protégé,
- B représente un radical dérivé d'une base purique ou pyrimidique de formule : 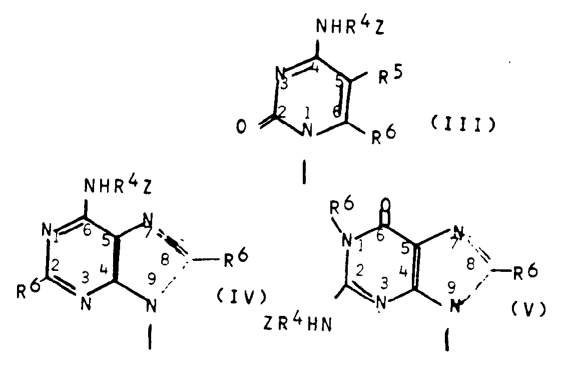 dans lesquelles R⁵ représente H, CH₃ ou R⁴Z, R⁶ représente un atome d'hydrogène ou le groupement R⁴Z et le radical de formule B est relié par le N en position 1 du radical de formule III ou en position 9 des radicaux de formule IV et V à la partie osidique,
- R⁴ représente une simple liaison ou un radical hydrocarboné choisi parmi les radicaux de formules :
-NH(CH₂)ₙ-X-(CH₂)ₚ-
-NH-(CH₂)ₙ-X-(CH₂)ₚ-NH-
-O-(CH₂)ₙ-X-(CH₂)ₚ-
-O-(CH₂)ₙ-X-(CH₂)ₚ-NH-
-S-(CH₂)ₙ-X-(CH₂)ₚ-
-S-(CH₂)ₙ-X-(CH₂)ₚ-NH-
-NHCO-(CH₂)ₙ-X-(CH₂)ₚ-
-NHCO-(CH₂)ₙ-X-(CH₂)ₚ-NH-
-NHSO₂-(CH₂)ₙ-X-(CH₂)ₚ-
-NHSO₂-(CH₂)ₙ-X-(CH₂)ₚ-NH-
-(CH₂)ₙ-X-(CH₂)ₚ-
-(CH₂)ₙ-X-(CH₂)ₚ-NH
-CO-(CH₂)ₙ-X-(CH₂)ₚ-
-CO-(CH₂)ₙ-X-(CH₂)ₚ-NH-
dans lesquelles n est un nombre entier allant de 1 à 6, p est égal à 0 ou est un nombre entier de 1 à 6, X représente CH₂, O, S, NH, CO, -HC=CH-, -(CH₂)ᵣZ, (CH₂)ᵣNHZ, 〉C=CH(CH₂)ᵣZ, 〉C=CH(CH₂)ᵣNHZ, 〉N(CH₂)ᵣZ, 〉N(CH₂)ᵣNHZ, 〉CH-O(CH₂)ᵣZ, 〉CH-O-(CH₂)ᵣNHZ, 〉HCS(CH₂)ᵣZ, 〉HCS(CH₂)ᵣNHZ avec r= 1 à 6 ;
- Z est un groupe protecteur utilisable en synthèse peptidique ou un radical dérivé d'une molécule de marquage choisie dans le groupe des molécules de marquage détectables directement et des molécules capables de recevoir un élément radioactif pour servir de marqueurs,
à condition que Z ne représente un groupe protecteur que lorsque R² est un support comportant un bras espaceur de liaison.

2. Dérivé de nucléoside selon la revendication 1, caractérisé en ce que la molécule de marquage détectable directement est une molécule capable de s'associer à l'avidine ou la streptavidine.

3. Dérivé selon la revendication 2, caractérisé en ce que Z est choisi parmi les radicaux de formule : 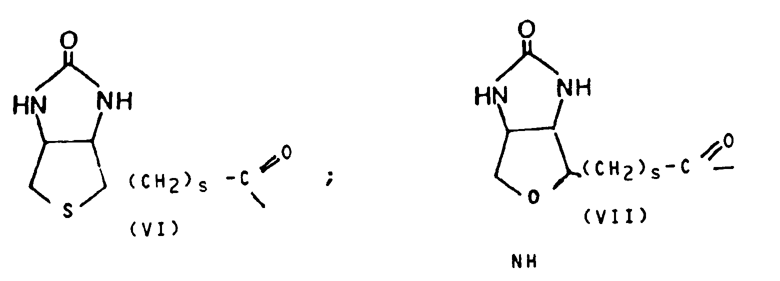 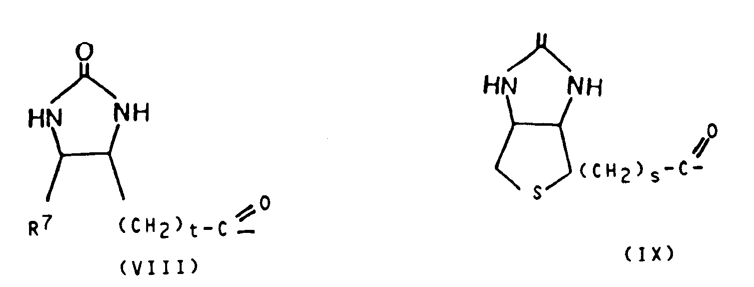 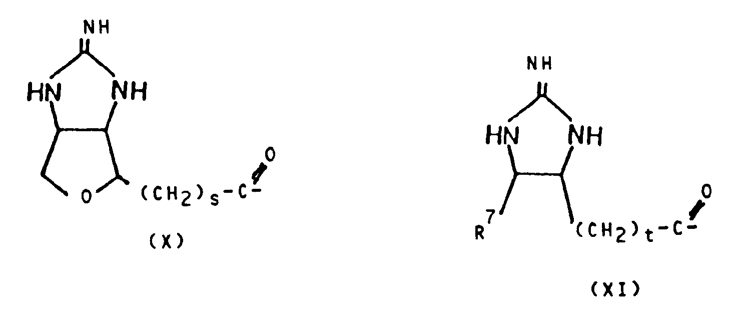 dans lesquelles
s= 4, 5, 6 ou 7
t= 3, 4, 5, 6 ou 7 et
R⁷ est un radical alkyle.

4. Dérivé de nucléoside selon la revendication 1, caractérisé en ce que la molécule de marquage détectable directement est une molécule pouvant donner lieu à une réaction du type antigène-anticorps.

5. Dérivé de nucléoside selon la revendication 4, caractérisé en ce que Z est un radical choisi parmi les radicaux de formules suivantes :

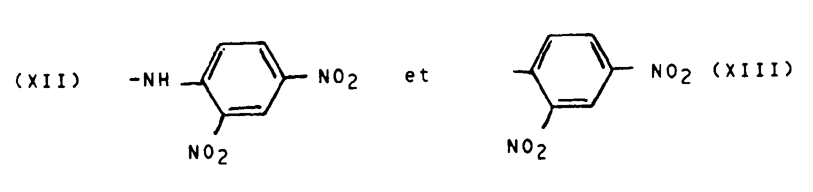

6. Dérivé de nucléoside selon la revendication 1, caractérisé en ce que la molécule de marquage détectable directement est une molécule possédant des propriétés fluorescentes.

7. Dérivé de nucléoside selon la revendication 6, caractérisé en ce que Z est un radical choisi parmi les radicaux suivants : 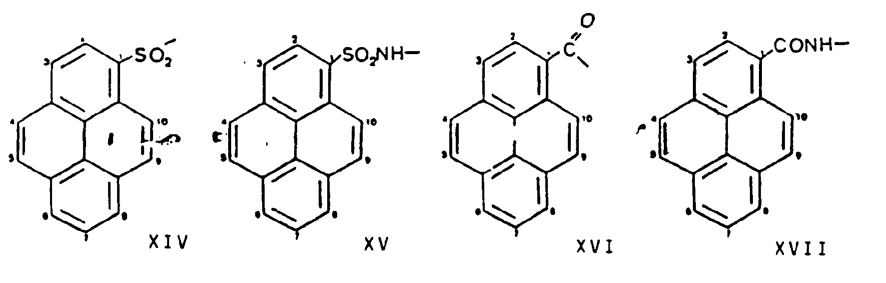 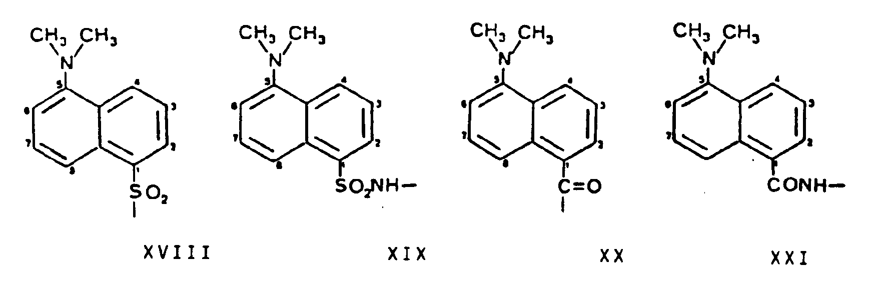 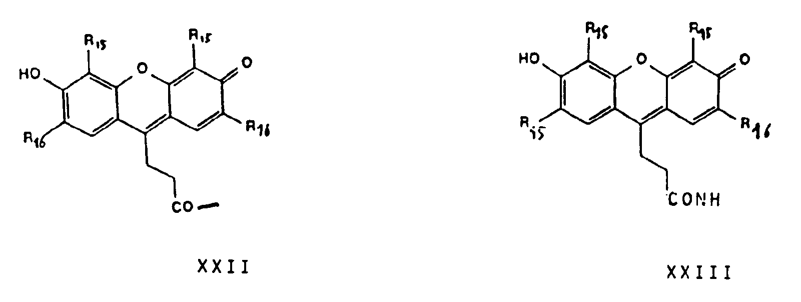 dans lesquels R¹⁵ et R¹⁶ qui peuvent être identiques ou différents sont un atome d'hydrogène ou un radical alkyle.

8. Dérivé de nucléoside selon la revendication 1, caractérisé en ce que la molécule capable de recevoir un élément radioactif est une molécule susceptible d'être marquée à l'iode.

9. Dérivé de nucléoside selon la revendication 8, caractérisé en ce que Z est choisi parmi les radicaux de formule : 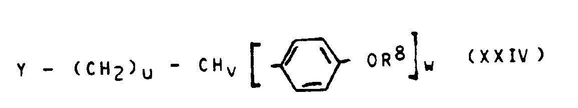 et 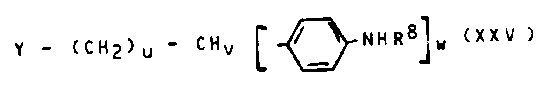 dans lesquelles :
u = 0 ou est un nombre entier de 1 à 6,
v = 0, 1 ou 2,
u = 3, 2, 1 avec v+W=3,
R⁸ représente un atome d'hydrogène ou un radical acyle, et
Y représente CH₂, HN, O, S, CO, SO₂, CONH, SO₂NH.

10. Dérivé de nucléoside selon la revendication 1, caractérisé en ce que B représente le radical de formule : 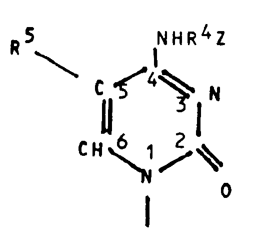 dans laquelle R⁵ représente H ou CH₃, R⁴ et Z ont la signification donnée dans la revendication 1.

11. Dérivé de nucléoside selon la revendication 10, caractérisé en ce que R⁴Z représente le radical de formule : (CH₂)₆- NHZ
dans laquelle Z est un radical de formule :

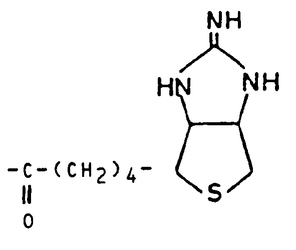

12. Dérivé selon l'une quelconque des revendications 10 et 11, caractérisé en ce que R⁴Z représente

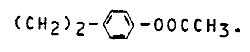

13. Dérivé selon l'une quelconque des revendications 10 à 12, caractérisé en ce que R¹ est un atome d'hydrogène ou le radical diméthoxytrityle.

14. Dérivé selon l'une quelconque des revendications 10 à 13, caractérisé en ce que R² est un radical phosphoré de formule :

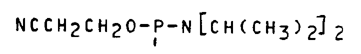

15. Dérivé selon l'une quelconque des revendications 10 et 13, caractérisé en ce que R² est le radical de formule OC(CH₂)₂ CONH 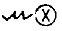 dans laquelle NH 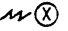 représente un support de silice ayant un bras espaceur aminé.

16. Procédé de synthèse d'oligonucléotides consistant à effectuer plusieurs cycles de condensation dans chacun desquels on condense un dérivé de nucléoside sur un dérivé de nucléoside ou sur un oligonucléotide, caractérisé en ce que l'un au moins des dérivés de nucléoside utilisé est un dérivé de nucléoside répondant à la formule : 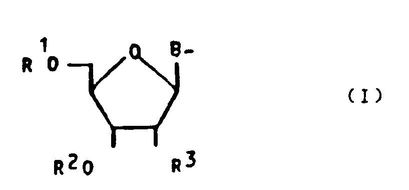 dans laquelle :
- R¹ représente un radical labile en milieu acide convenant à la synthèse nucléotidique,
- R² représente un radical fonctionnel adapté à ia synthèse nucléotidique, ou un support utilisable en synthèse nucléotidique comportant un bras espaceur de liaison avec l'atome d'oxygène,
- R³ représente un atome d'hydrogène, le radical hydroxyle ou un radical hydroxyle protégé, et
- B, a la signification donnée dans la revendication 1.

17. Oligonucléotide marqué comportant une séquence de différents nucléotides, caractérisé en ce qu'au moins deux nucléotides de la séquence sont marqués par des marqueurs différents.

18. Oligonucléotide marqué comportant une séquence de différents nucléotides, caractérisé en ce qu'au moins l'un des nucléotides de la séquence est marqué par un radical choisi parmi
- les radicaux de formules : 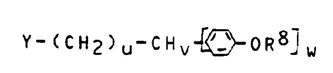 ou 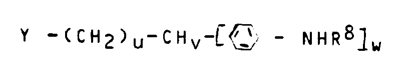 dans lesquelles
u = 0 ou est un nombre entier de 1 à 6,
v = 0, 1 ou 2,
w = 3, 2ou 1 avec v+w=3,
R⁸ représente un atome d'hydrogène ou un radical acyle, et
Y représente CH₂, NH, O, S, CO, SO₂, CONH, ou SO₂NH,
- les radicaux de formule : 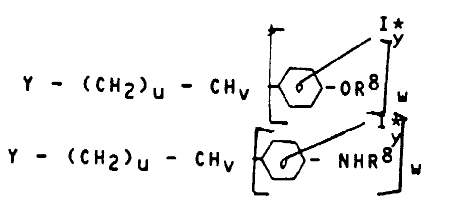 dans lesquelles R⁸ est un atome d'hydrogène et u, v, w, R⁸ et Y ont la signification donnée ci-dessus, y=1, 2, 3 ou 4 et I* est un atome d'iode radioactif ;
- les radicaux de formule : 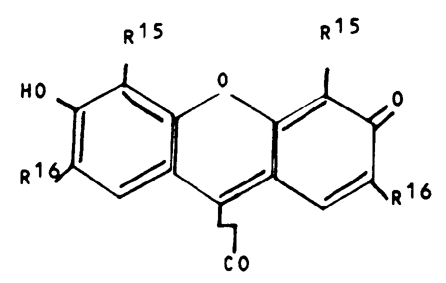 dans laquelle R¹⁵ et R¹⁶ qui peuvent être identiques ou différents, représentent un atome d'hydrogène ou un radical alkyle ; et
- les radicaux pyrényle de formule :

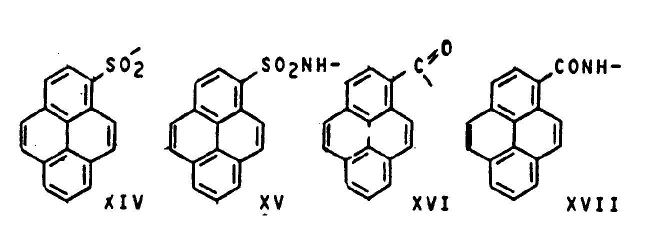

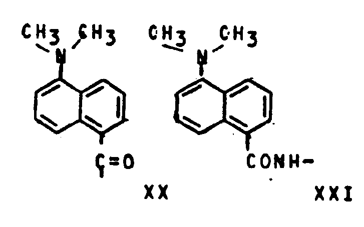

19. Oligonucléotide marqué comportant une séquence de différents nucléotides, caractérisé en ce qu'au moins 5 nucléotides situés à au moins l'une des extrêmités de la séquence sont marqués par des molécules de marquage ou des précurseurs de ces molécules.

20. Oligonucléotide marqué comportant une séquence de différents nucléotides, caractérisé en ce qu'au moins deux nucléotides voisins situés à l'extrémité 5' de la séquence sont marqués par des molécules de marquage ou des précurseurs de ces molécules.

## Patentansprüche

1. Nucleosidderivat, das zur Synthese von markierten Oligonucleotiden verwendbar ist, dadurch gekennzeichnet, daß es der Formel 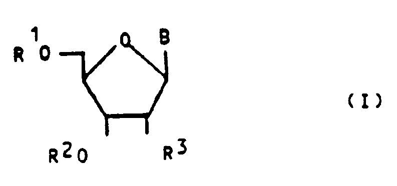 entspricht, in der
- R¹ ein Wasserstoffatom oder eine im sauren Medium labile, zur Nucleotidsynthese geeignete Gruppe bedeutet,
- R² ein Wasserstoffatom, eine Schutzgruppe, eine der Oligonucleotidsynthese angepaßte funktionelle Gruppe oder einen zur Oligonucleotidsynthese verwendbaren Träger bedeutet, der einen Linker zur Verbindung mit dem Sauerstoffatom besitzt,
- R³ ein Wasserstoffatom, die Hydroxylgruppe oder eine geschützte Hydroxylgruppe bedeutet,
- B einen Rest bedeutet, der von einer Purin- oder Pyrimidinbase der Formel 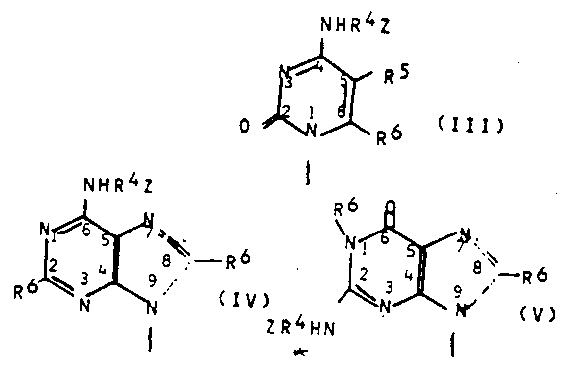 abgeleitet ist, worin R⁵ für H, CH₃ oder R⁴Z steht, R⁶ ein Wasserstoffatom oder den Rest R⁴Z bedeutet und der Rest B durch den Stickstoff in Position 1 des Restes der Formel III oder in Position 9 der Reste der Formeln IV und V mit dem Zuckerteil verbunden ist,
- R⁴ eine einfache Bindung oder einen kohlenwasserstoffhaltigen Rest bedeutet, ausgewählt aus den Resten der Formeln
-NH(CH₂)ₙ-X-(CH₂)ₚ-
-NH-(CH₂)ₙ-X-(CH₂)ₚ-NH-
-O-(CH₂)ₙ-X-(CH₂)ₚ-
-O-(CH₂)ₙ-X-(CH₂)ₚ-NH-
-S-(CH₂)ₙ-X-(CH₂)ₚ-
-S-(CH₂)ₙ-X-(CH₂)ₚ-NH-
-NHCO-(CH₂)ₙ-X-(CH₂)ₚ-
-NHCO-(CH₂)ₙ-X-(CH₂)ₚ-NH-
-NHSO₂-(CH₂)ₙ-X-(CH₂)ₚ-
-NHSO₂-(CH₂)ₙ-X-(CH₂)ₚ-NH-
-(CH₂)ₙ-X-(CH₂)ₚ-
-(CH₂)ₙ-X-(CH₂)ₚ-NH-
-CO-(CH₂)ₙ-X-(CH₂)ₚ-
-CO-(CH₂)ₙ-X-(CH₂)ₚ-NH- ,
worin n eine ganze Zahl von 1 bis 6 ist, p gleich Null oder eine ganze Zahl von 1 bis 6 ist, X für CH₂, S, NH, CO, -CH=CH-, -(CH₂)ᵣZ, -(CH₂)ᵣNHZ, 〉C=CH(CH₂)ᵣZ, 〉C=CH(CH₂)ᵣNHZ, 〉N(CH₂)ᵣZ, 〉N(CH₂)ᵣNHZ, 〉CH-O(CH₂)ᵣZ, 〉CH-O(CH₂)ᵣNHZ, 〉HCS(CH₂)ᵣZ, 〉HCS(CH₂)ᵣNHZ mit r von 1 bis 6 steht,
- Z eine bei der Peptidsynthese verwendbare Schutzgruppe oder ein Rest ist, der sich von einem Markierungsmolekül ableitet, das gewählt ist in der Gruppe der direkt detektierbaren Markierungsmoleküle und der Moleküle, die ein radioaktives Element aufnehmen können, um als Marker zu dienen, unter der Bedingung, daß Z nur dann eine Schutzgruppe bedeutet, wenn R² ein Träger ist, der einen Linker besitzt.

2. Nucleosidderivat nach Anspruch 1, dadurch gekennzeichnet, daß das direkt detektierbare Markierungsmolekül ein Molekül ist, das zur Assoziation an Avidin oder Streptavidin in der Lage ist.

3. Derivat nach Anspruch 2, dadurch gekennzeichnet, daß Z unter den Gruppen der Formeln 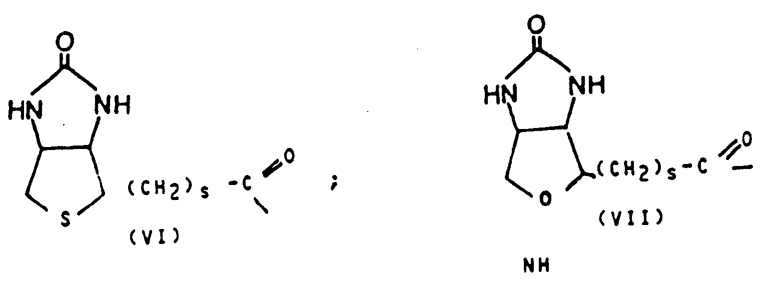 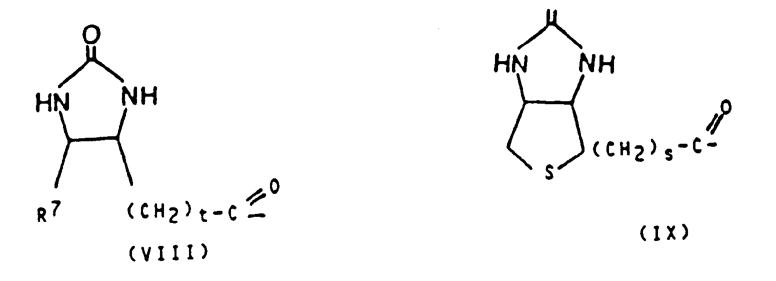 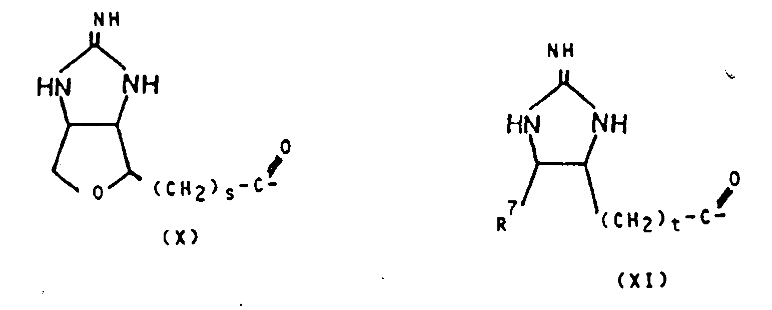 gewählt ist, worin
s = 4, 5, 6 oder 7,
t = 3, 4, 5, 6 oder 7,
und R⁷ ein Alkylrest ist.

4. Nucleosidderivat nach Anspruch 1, dadurch gekennzeichnet, daß das direkt detektierbare Markierungsmolekül ein Molekül ist, das zu einer Reaktion vom Antigen-Antikörper-Typ Anlaß geben kann.

5. Nucleosidderivat nach Anspruch 4, dadurch gekennzeichnet, daß Z ein Rest ist, der unter den Resten der folgenden Formeln ausgewählt ist:

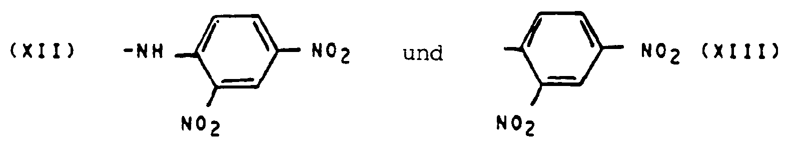

6. Nucleosidderivat nach Anspruch 1, dadurch gekennzeichnet, daß das direkt detektierbare Markierungsmolekül Fluoreszenzeigenschaften besitzt.

7. Nucleosidderivat nach Anspruch 6, dadurch gekennzeichnet, daß Z ein Rest ist, der unter den folgenden Resten ausgewählt ist: 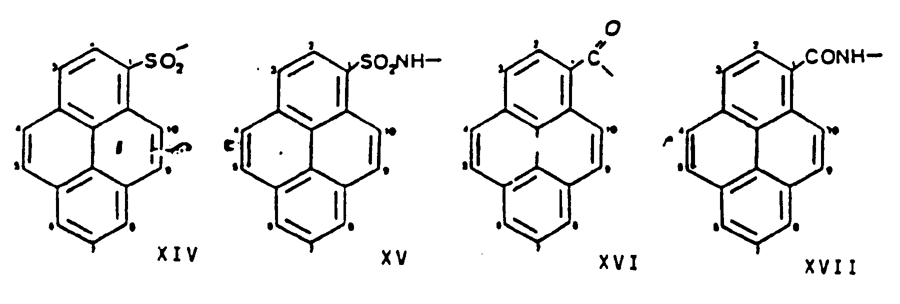 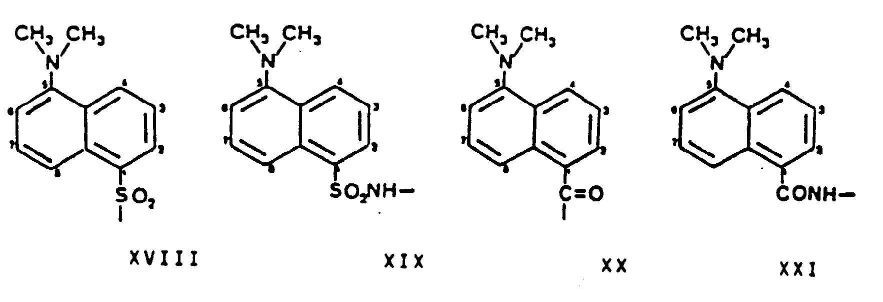 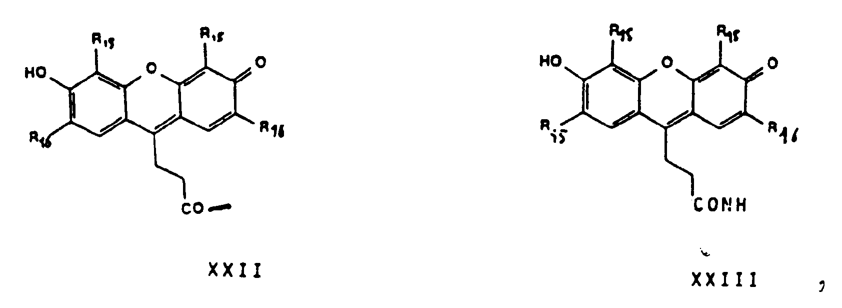 worin R¹⁵ und R¹⁶, die gleich oder verschieden sein können, ein Wasserstoffatom oder einen Alkylrest bedeuten.

8. Nucleosidderivat nach Anspruch 1, dadurch gekennzeichnet, daß das Molekül, das die Fähigkeit besitzt, ein radioaktives Element aufzunehmen, ein Molekül ist, das mit Jod markiert werden kann.

9. Nucleosidderivat nach Anspruch 8, dadurch gekennzeichnet, daß Z unter den Resten der Formeln 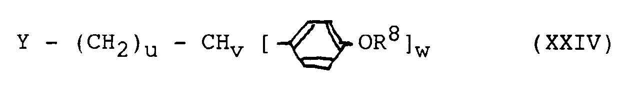 und 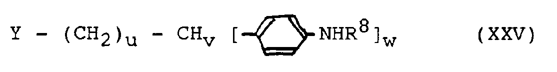 ausgewählt ist, worin
u = 0 oder eine ganze Zahl von 1 bis 6,
v = 0, 1 oder 2,
w = 3, 2, 1 mit v + w = 3,
R⁸ ein Wasserstoffatom oder einen Acylrest darstellt und Y für CH₂, HN, O, S, CO, SO₂, CONH, SO₂NH steht.

10. Nucleosidderivat nach Anspruch 1, dadurch gekennzeichnet, daß B den Rest der Formel 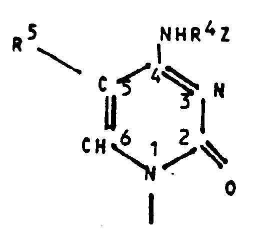 darstellt, worin R⁵ für H oder CH₃ steht, R⁴ und Z die in Anspruch 1 gegebene Bedeutung haben.

11. Nucleosidderivat nach Anspruch 10, dadurch gekennzeichnet, daß R⁴Z den Rest der Formel (CH₂)₆-NHZ bedeutet, worin Z ein Rest der Formel 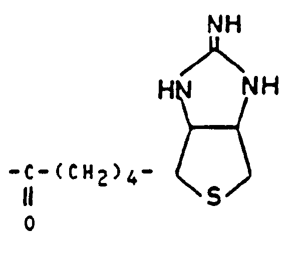 ist.

12. Derivat nach einem der Ansprüche 10 und 11, dadurch gekennzeichnet, daß R⁴Z für 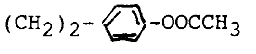 steht.

13. Derivat nach einem der Ansprüche 10 bis 12, dadurch gekennzeichnet, daß R¹ ein Wasserstoffatom oder die Dimethoxytritylgruppe ist.

14. Derivat nach einem der Ansprüche 10 bis 13, dadurch gekennzeichnet, daß R² eine phosphorhaltige Gruppe der Formel 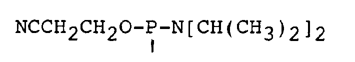 ist.

15. Derivat nach einem der Ansprüche 10 und 13, dadurch gekennzeichnet, daß R² die Gruppe der Formel OC(CH₂)₂-CONH 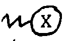 ist, in der NH 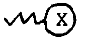 einen Siliciumdioxidträger bedeutet, der einen aminierten Linker besitzt.

16. Verfahren zur Synthese von Oligonucleotiden, das darin besteht, mehrere Kondensationszyklen durchzuführen, in deren jedem man ein Nucleosidderivat mit einem Nucleosidderivat oder einem Oligonucleotid kondensiert, dadurch gekennzeichnet, daß wenigstens eines der verwendeten Nucleosidderivate ein der Formel 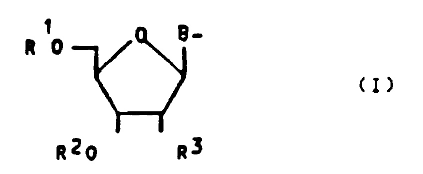 entsprechendes Nucleosidderivat ist, worin
- R¹ eine im sauren Medium labile, zur Nucleotidsynthese geeignete Gruppe bedeutet,
- R² eine der Nucleotidsynthese angepaßte funktionelle Gruppe oder einen bei der Nucleotidsynthese verwendbaren Träger bedeutet, der einen Linker zur Bindung mit dem Sauerstoffatom besitzt,
- R³ ein Wasserstoffatom, die Hydroxylgruppe oder eine geschützte Hydroxylgruppe bedeutet, und
- B die in Anspruch 1 gegebene Bedeutung hat.

17. Markiertes, eine Sequenz verschiedener Nucleotide aufweisendes Oligonucleotid, dadurch gekennzeichnet, daß wenigstens zwei Nucleotide der Sequenz mit unterschiedlichen Markern markiert sind.

18. Markiertes, eine Sequenz verschiedener Nucleotide aufweisendes Oligonucleotid, dadurch gekennzeichnet, daß wenigstens eines der Nucleotide der Sequenz durch einen Rest markiert ist, der ausgewählt ist unter
- den Resten der Formeln 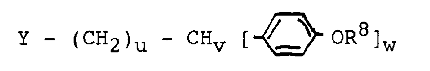 oder 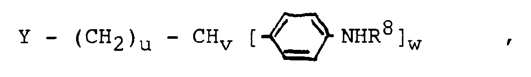 worin u = 0 oder eine ganze Zahl von 1 bis 6,
v = 0, 1 oder 2,
w = 3, 2, 1 mit v + w = 3,
R⁸ ein Wasserstoffatom oder einen Acylrest darstellt, und Y für CH₂, HN, O, S, CO, SO₂, CONH, SO₂NH steht,
- den Resten der Formel 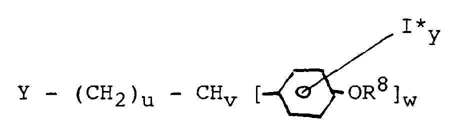 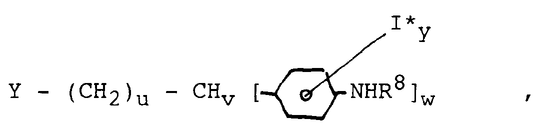 worin R⁸ ein Wasserstoffatom ist und u, v, w, R⁸ und Y die oben gegebene Bedeutung haben, y = 1, 2, 3 oder 4, und I* ein radioaktives Jodatom ist,
- den Resten der Formel 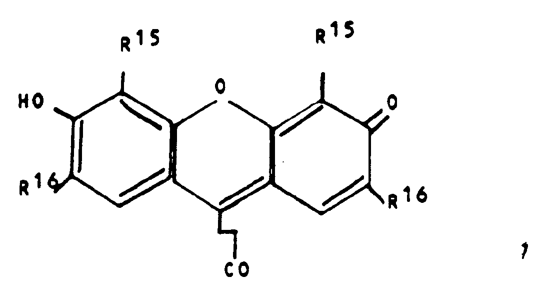 worin R¹⁵ und R¹⁶, die gleich oder verschieden sein können, ein Wasserstoffatom oder einen Alkylrest bedeuten, und
- den Pyrenylresten der Formel

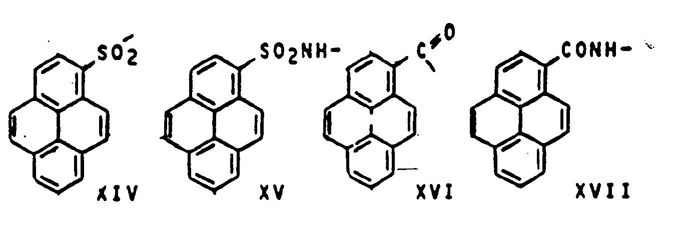

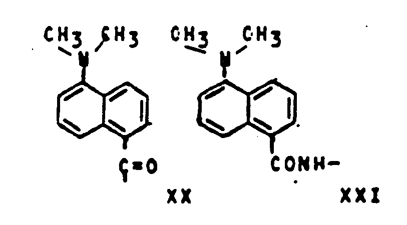

19. Markiertes, eine Sequenz verschiedener Nucleotide aufweisendes Oligonucleotid, dadurch gekennzeichnet, daß wenigstens fünf Nucleotide, die sich an wenigstens einem der Enden der Sequenz befinden, durch Markierungsmoleküle oder durch Vorläufer dieser Moleküle markiert sind.

20. Markiertes, eine Sequenz verschiedener Nucleotide aufweisendes Oligonucleotid, dadurch gekennzeichnet, daß wenigstens zwei benachbarte Nucleotide, die sich am Ende 5' der Sequenz befinden, durch Markierungsmoleküle oder durch Vorläufer dieser Moleküle markiert sind.

## Claims

1. Nucleoside derivative usable for the synthesis of marked oligonucleotides characterized in that it complies with the formula: 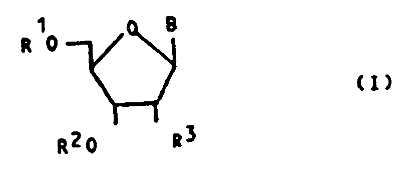 in which:
- R¹ represents a hydrogen atom or an unstable radical in an acid medium appropriate for nucleotide synthesis,
- R² represents a hydrogen atom, a protective radical, a functional radical suitable for oligonucleotide synthesis, or a support usable for oligonucleotide synthesis having a bond spacing arm with the oxygen atom,
- R³ represents a hydrogen atom, the hydroxyl radical or a protected hydroxyl radical,
- B represents a radical derived from a purine or pyrimidine base of formula: 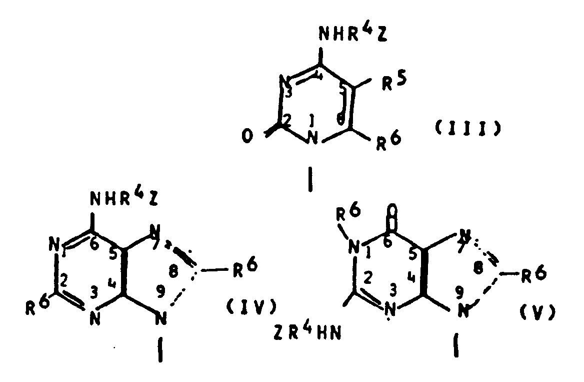 in which R⁵ represents H, CH₃ or R⁴Z, R⁶ represents a hydrogen atom or the R⁴Z group and the radical of formula B is linked by N in the 1-position of the radical of formula III or in the 9-position of the radicals of formulas IV and V to the oside part,
- R⁴ represents a single bond or a hydrocarbon radical chosen from among the radicals of formulas:
-NH(CH₂)ₙ-X-(CH₂)ₚ-
-NH-(CH₂)ₙ-X-(CH₂)ₚ-NH-
-O-(CH₂)ₙ-X-(CH₂)ₚ-
-O-(CH₂)ₙ-X-(CH₂)ₚ-NH-
-S-(CH₂)ₙ-X-(CH₂)ₚ-
-S-(CH₂)ₙ-X-(CH₂)ₚ-NH-
-NHCO-(CH₂)ₙ-X-(CH₂)ₚ
-NHCO-(CH₂)ₙ-X-(CH₂)ₚ-NH-
-NHSO₂-(CH₂)ₙ-X-(CH₂)ₚ-
-NHSO₂-(CH₂)ₙ-X-(CH₂)ₚ-NH-
-(CH₂)ₙ-X-(CH₂)ₚ-
-(CH₂)ₙ-X-(CH₂)ₚ-NH
-CO-(CH₂)ₙ-X-(CH₂)ₚ-
-CO-(CH₂)ₙ-X-(CH₂)ₚ-NH-
in which n is an integer between 1 and 6, p is equal to 0 or is an integer from 1 to 6, X represents CH₂, O, S, NH, CO, HC=CH-, -(CH₂)ᵣZ, (CH₂)ᵣNHZ, 〉C=CH(CH₂)ᵣZ, 〉C=CH(CH₂)ᵣNHZ, 〉(CH₂)ᵣZ, 〉N(CH₂)ᵣNHZ, 〉CH-O(CH₂)ᵣZ, 〉CH-O-(CH₂)_{R}NHZ, 〉HCS(CH₂)ᵣZ, 〉HCS(CH₂)ᵣNHZ with r = 1 to 6.
- Z is a protective group usable in peptide synthesis or a radical derived from a marking molecule chosen from the group of directly detectable marking molecules and molecules able to receive a radioactive element to serve as markers, provided that Z only represents a protective group when R² is a support having a bond spacing arm.

2. Nucleoside derivative according to claim 1, characterized in that the directly detectable marking molecule is a molecule which can be combined with avidin or streptavidin.

3. Derivative according to claim 2, characterized in that Z is chosen from among the radicals of formula: 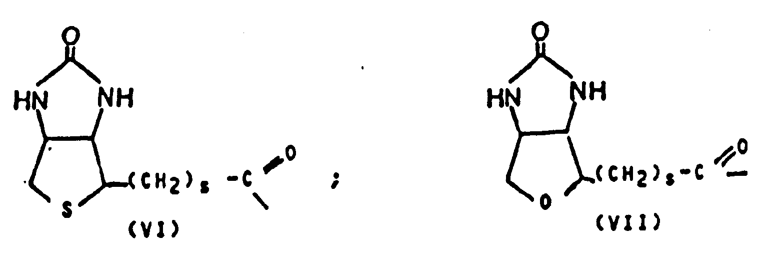 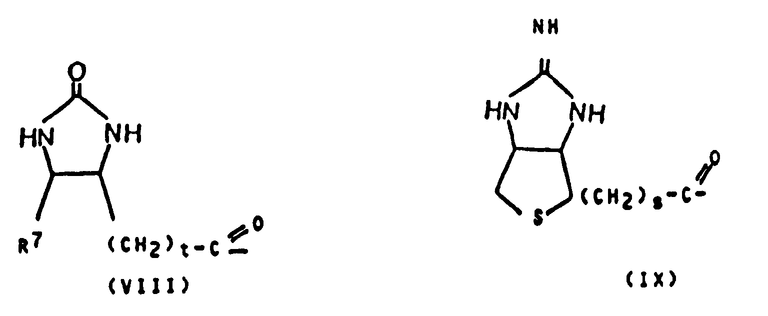 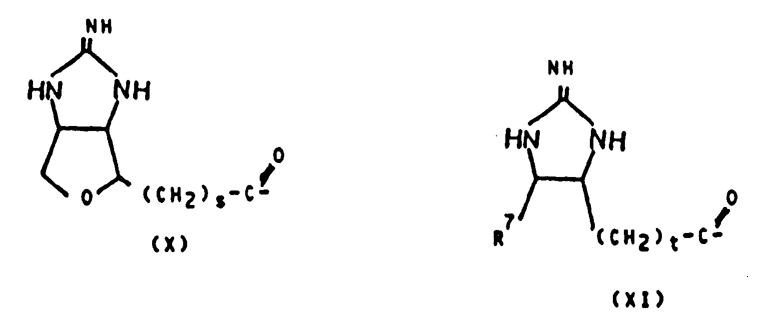 in which
s = 4, 5, 6 or 7
t = 3, 4, 5, 6 or 7 and
R⁷ is an alkyl radical.

4. Nucleoside derivative according to claim 1, characterized in that the directly detectable marking molecule is a molecule which can give rise to a reaction of the antigen - antibody type.

5. Nucleoside derivative according to claim 4, characterized in that Z is a radical chosen from among the radical of formulas:

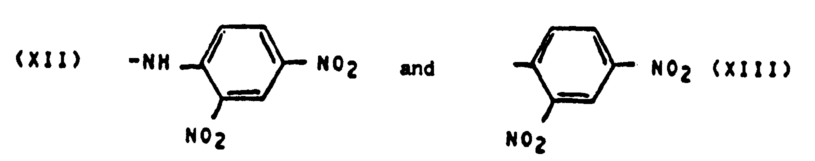

6. Nucleoside derivative according to claim 1, characterized in that the directly detectable marking molecule is a molecule having fluorescent properties.

7. Nucleoside derivative according to claim 6, characterized in that Z is a radical chosen from among the following radicals: 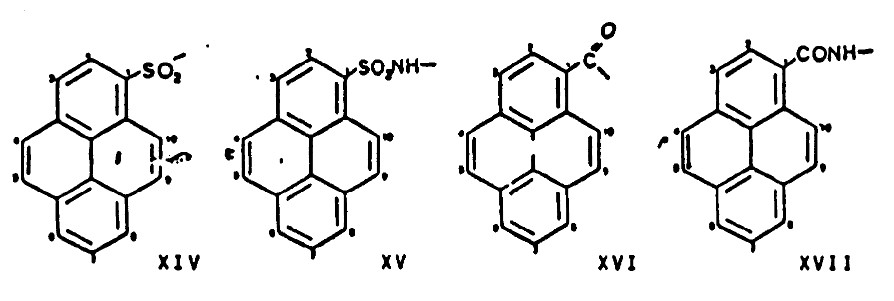 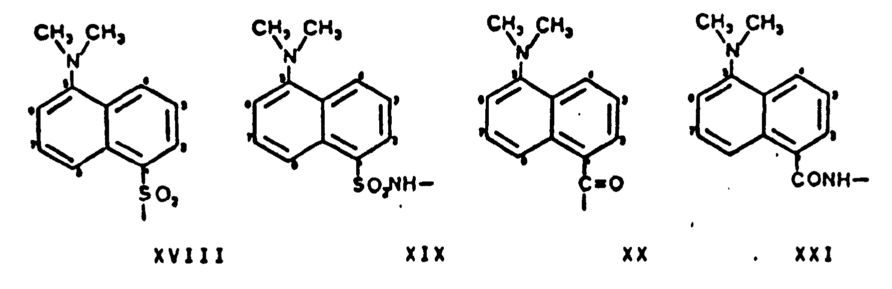 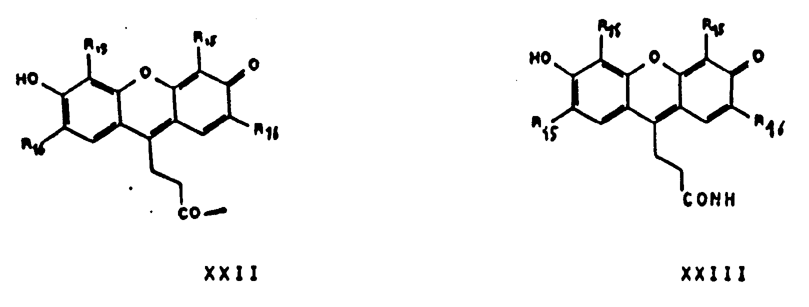 are a hydrogen atom or an alkyl radical.

8. Nucleoside derivative according to claim 1, characterized in that the molecule able to receive a radioactive element is a molecule which can be marked with iodine.

9. Nucleoside derivative according to claim 8, characterized in that Z is chosen from among the radicals of formula: 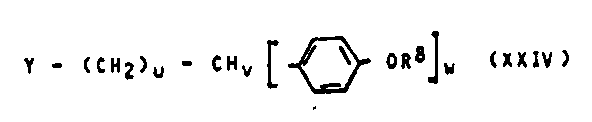 and 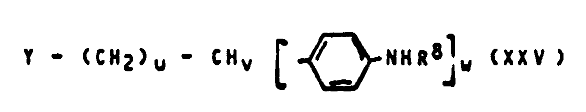 in which:
u = 0 or an integer from 1 to 6,
v = 0, 1 or 2,
w = 3, 2, 1 with v+W=3,
R⁸ represents a hydrogen atom or an acyl radical and Y represents CH₂, HN, O, S, CO, SO₂, CONH, SO₂NH.

10. Nucleoside derivative according to claim 1, characterized in that B represents the radical of formula: 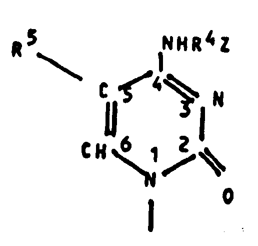 in which R⁶ represents H or CH₃, R⁴ and Z have the meanings given in claim 1.

11. Nucleoside derivative according to claim 10, characterized in that R⁴Z represents the radical of formula: (CH₂)₆-NHZ in which Z is a radical of formula:

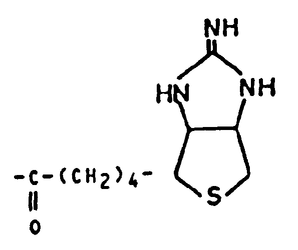

12. Derivative according to any one of the claims 10 and 11, characterized in that R⁴Z represents

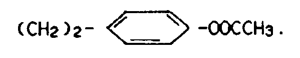

13. Derivative according to any one of the claims 10 to 12, characterized in that R¹ is a hydrogen atom or the dimethoxytrityl radical.

14. Derivative according to any one of the claims 10 to 13, characterized in that R² is a phosphorus radical of formula:

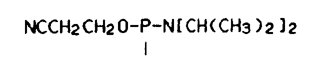

15. Derivative according to any one of the claims 10 and 13, characterized in that R² is the radical of formula OC(CH₂)₂ CONH 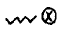 in which NH 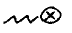 represents a silica support having an amino spacing arm.

16. Process for oligonucleotide synthesis consisting of carrying out several condensation cycles, in each of which a nucleoside derivative is condensed on a nucleoside derivative or on an oligonucleotide, characterized in that at least one of the nucleoside derivatives used is a nucleoside derivative complying with the formula: 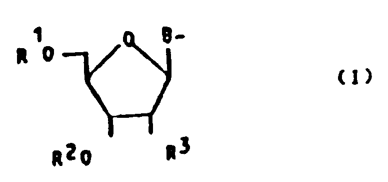 in which:
- R¹ represents an unstable radical in the acid medium suitable for nucleotide synthesis,
- R² represents a functional radical suitable for nucleotide synthesis, or a support usable in nucleotide synthesis having a bond spacing arm with the oxygen atom,
- R³ represents a hydrogen atom, the hydroxyl radical or a protected hydroxyl radical and
- B has the meaning given in claim 1.

17. Marked oligonucleotide having a sequence of different nucleotides, characterized in that at least two nucleotides of the sequence are marked by different markers.

18. Marked oligonucleotide having a sequence of different nucleotides, characterized in that at least one of the nucleotides is marked by a radical chosen from among radicals of formula 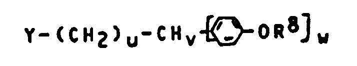 or 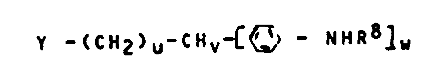 in which
u = 0 or an integer from 1 to 6,
v = 0, 1 or 2,
W = 3, 2 or 1 with v+w=3,
R⁸ represents a hydrogen atom or an acyl radical, and
Y represents CH₂, NH, O, S, CO, SO₂, CONH, or SO₂NH,
- the radicals of formula: 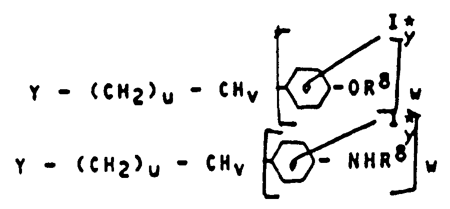 in which R⁸ is a hydrogen atom and u, v, w and Y have the meanings given hereinbefore, y=1, 2, 3 or 4 and I* is a radioactive iodine atom;
- the radicals of formula: 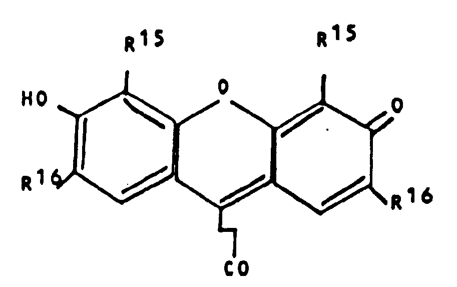 in which R¹⁵ and R¹⁶, which can be the same or different represent a hydrogen atom or an alkyl radical; and
- the pyrenyl radicals of formula:

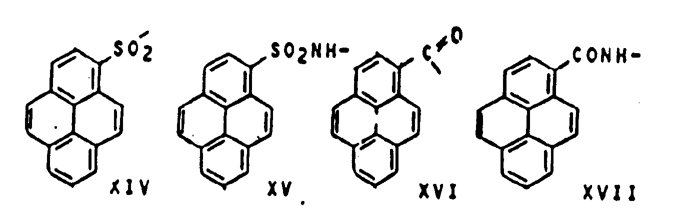

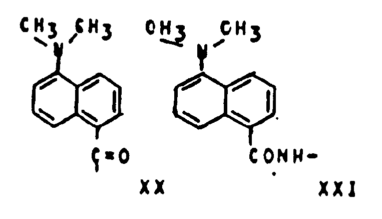

19. Marked oligonucleotide having a sequence of different nucleotides, characterized in that at least 5 nucleotides located at at least one of the ends of the sequence are marked by marking molecules or precursors of said molecules.

20. Marked oligonucleotide having a sequence of different nucleotides, characterized in that at least two adjacent nucleotides located at the 5' end of the sequence are marked by marking molecules or precursors of said molecules.
